(19) Europäisches Patentamt

European Patent Office

Office européen des brevets

(11) **EP 3 567 594 B1**

(12) **EUROPEAN PATENT SPECIFICATION**

(45) Date of publication and mention
of the grant of the patent:
**07.07.2021 Bulletin 2021/27**

(51) Int Cl.:
**G16H 20/17** *(2018.01)* **G16H 50/20** *(2018.01)*
**A61M 5/00** *(2006.01)*

(21) Application number: **18171649.9**

(22) Date of filing: **09.05.2018**

(54) **DIABETES MANAGEMENT SYSTEM WITH DYNAMIC SELECTION OF PREDICTION LOGIC**

DIABETISVERWALTUNGSSYSTEM MIT DYNAMISCHER AUSWAHL DER VORHERSAGELOGIK

SYSTÈME DE GESTION DU DIABÈTE AVEC SÉLECTION DYNAMIQUE D'UNE LOGIQUE DE PRÉDICTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**13.11.2019 Bulletin 2019/46**

(73) Proprietor: **SAP SE
69190 Walldorf (DE)**

(72) Inventors:
• **KOPP, Ulrich
69160 Walldorf (DE)**

• **RIEGEL, Lukas
69190 Walldorf (DE)**

(74) Representative: **Richardt Patentanwälte PartG
mbB
Wilhelmstraße 7
65185 Wiesbaden (DE)**

(56) References cited:
**EP-A1- 2 572 740 WO-A1-2014/011740
WO-A1-2015/066522 US-A1- 2016 378 945
US-A1- 2017 049 963 US-A1- 2017 177 822
US-A1- 2017 286 638 US-A1- 2017 311 903**

## Description

## Field of the invention

The invention relates to the field of diabetes management systems, and more particularly to diabetes management systems comprising a portable computing device, a server computer, an insulin pump and other data collection units.

## Background and related art

Diabetes mellitus is a disorder in which the pancreas cannot create sufficient insulin and/or in which insulin is not effective. In the diabetic state, the victim suffers from high blood sugar, which can cause an array of physiological derangements such as, for example, kidney failure, skin ulcers, or bleeding into the vitreous of the eye. A hypoglycemic reaction (low blood sugar) can be induced by an inadvertent overdose of insulin, or after a normal dose of insulin or glucose-lowering agent accompanied by extraordinary exercise or insufficient food intake.

Conventionally, a person with diabetes carries a self-monitoring blood glucose (SMBG) monitor, which typically requires uncomfortable finger pricks to obtain blood samples for measurement. Due to the lack of comfort and convenience associated with finger pricks, a person with diabetes normally only measures his or her glucose levels two to four times per day. The time intervals between two measurements are often too far apart and a person with diabetes may thus find out too late of a hyperglycemic or hypoglycemic condition.

Another device that some diabetics use to monitor their blood glucose is a continuous analyte sensor, in particular a continuous glucose meter. A continuous analyte sensor typically includes a sensor that is placed subcutaneously, transdermally (e.g., trans cutaneously), or intravascularly. The sensor measures the concentration of a given analyte within the body, and generates a raw signal that is transmitted to electronics associated with the sensor. The raw signal is converted into an output value that is displayed on a display.

There are many products available to help maintain records and analyze trends related to diabetes care. Some diabetes management software is free to download or can be used on-line, while others are for sale (or shareware). Some are simple and easy to use for a single user, while others have very powerful features that support many users in a clinical setting. Some are delivered together with a particular hardware device, e.g. a glucose meter or an insulin pump, others are provided as software-only solution.

US 2014/0012511 A1 describes a diabetes management method combining the capabilities of a smartphone with information from a continuous glucose monitor and additional sensors to provide diabetics patients with more information than the continuous glucose monitor can provide by itself for improving the health of the patient. A smartphone comprises an auxiliary interface adapted to sense various physiological parameters of a patient, e.g. skin temperature, heart rate, and others. However, a smart phone comprising all the sensors mentioned in this document is not yet available in the market and is appears questionable if it ever will be: each sensor increases the price of the smartphone, and the number of customers lost by a higher price of a sensor-rich smartphone may outweigh the number of sensor-rich smart phones bought by diabetes patients. Moreover, multi-sensor smartphones are special-purpose products which may not correspond to the type of smartphone preferred by a user for non-disease-related reasons, e.g. style, design, operating system (Apple, Android or Windows) and which are inflexible as the number and type of sensors integrated in a smartphone can typically not be changed by the end user.

US patent application US 2017/049963 A1 (Varsavsky Andrea et al.), 2017-02-23, describes various medical devices and related patient management systems and parameter modeling methods. An exemplary method of operating a sensing device associated with a patient involves obtaining current operational context information associated with the sensing device, obtaining a parameter model associated with the patient, calculating a current parameter value based on the parameter model and the current operational context information, obtaining one or more signals from a sensing element configured to measure a condition in a body of the patient, and providing an output that is influenced by the calculated current parameter value and the one or more signals.

US patent application US 2017/311 903 A1 (Davis Anna Leigh), 2017-11-02, describes systems and methods that provide smart alerts to users, e.g., alerts to users about diabetic states that are only provided when it makes sense to do so, e.g., when the system can predict or estimate that the user is not already cognitively aware of their current condition, e.g., particularly where the current condition is a diabetic state warranting attention. In this way, the alert or alarm is personalized and made particularly effective for that user. Such systems and methods still alert the user when action is necessary, or provide a response to a missed holus or a need for correction, hut do not alert when action is unnecessary.

## Summary

It is an objective of the present invention to provide an improved method, system and computer program product as specified in the independent claims. Embodiments of the invention are given in the dependent claims. Embodiments of the present invention can be freely combined with each other if they are not mutually exclusive.

In one aspect, the invention relates to a system for diabetes disease management.

The system comprises a battery powered portable computing device comprising a client application. The portable computing device is assigned to a patient with diabetes. The portable computing device is operatively coupled to a first and other data collection units. Each data collection unit is configured to collect data of a respective data type. The data is related to the patient.

The system further comprises a server computer comprising a server application. The server application is interoperable with the client application and implements and maintains a plurality of different prediction logics. Each prediction logic is configured for remotely computing a prediction of a future physiologic state of the patient and/or for computing a prediction of a treatment suggestion for the patient.

The client application is configured for receiving first data from the first data collection unit. The first data collection unit comprises a glucose meter currently coupled to the portable computing device. Upon receiving the first data, the client application tries to receive other data from the other data collection units currently coupled to the portable computing device,

The server application is configured for receiving current patient data from the client application via a network. The received current patient data comprises data collected by the first data and the other data collection units. The server application is further configured for identifying the glucose meter and the other data collection units having collected the current patient data; for dynamically determining a number and type of the identified other data collection units; for automatically identifying and selecting a particular one out of the plurality of prediction logics as a function of at least the number and type of the identified one or more data collection units, the one identified prediction logic being adapted to use the totality of the received current patient data as input; and for applying the identified prediction logic at least on the received current patient data and on a history of the patient's patient data for computing the prediction. The server application is further configured for returning the prediction to the client application via the network.

The client application is further configured for sending a command to an insulin pump of the patient. The command causes the insulin pump to inject an amount of insulin to the patient that corresponds to a basal insulin rate specified in the prediction.

Each of the other data collection units is selected from a group comprising:

- a motion tracking sensor;
- an ECG (electrocardiogram) device, the data collected by the ECG device being physiological measurement data, in particular an electrocardiogram and/or data values derived therefrom;
- a respirometer, the data collected by the respirometer being physiological measurement data, in particular a rate of oxygen and/or carbon dioxide exchange;
- a scale, the data collected by the scale being the body weight of the patient;
- a thermometer, the data collected by the thermometer being a patient-proximal temperature, the patient-proximal temperature being the body temperature of the patient or being the temperature of the air surrounding the patient;
- an insulin pump logging unit, the data collected by the insulin pump logging unit being the time and/or amount of insulin applied to the patient.

For example, the portable computing device can be a tablet computer or a notebook. According to preferred embodiments, the portable computing device is a smartphone or a smart watch.

Said features may be advantageous as a system is provided that may be much more flexible than known diabetes management systems in respect to the number and types of sensors whose data is used for diabetes management. The prediction logic used for predicting the future health state or a recommended treatment option for a patient does not require or expect to receive a defined set of data values provided by a defined set of sensors. Rather, the server application maintains a plurality of different prediction logics, e.g. different subprograms, sub-routines or rule-based heuristics, and dynamically identifies and uses the one of these different prediction logics which is able to make best use of the available, currently collected patient data. This may be highly beneficial in the context of digital diabetes management: a smartphone alone may not comprise all sensors which could potentially provide useful measurement data for predicting a future state of a patient. However, applicant has observed that many of the digital "gadgets" which are used today by many people irrespective of whether or not they suffer from diabetes, may provide a good data basis for prediction. However, applicant has observed that the diabetes management programs currently available are not able to deal with the data provided by these digital gadgets such as wearable thermometers or ECG devices, because the availability of said data sources is highly variable.

For example, a patient may use an ECG device sensor sporadically about once a week, may use a gyrometer and a thermometer contained in a fitness bracelet every weekday morning at about 7:30 am and may implicitly use the GPS sensor contained in his or her smartphone the whole day. At least upon each moment when the client application device receives new data from a glucose meter of the patient, the client application may be configured to try receiving measurement data also from all other currently coupled data collection units. Thus, although any of said data collection units (GPS sensor and ECG indicating physical activity and thus glucose consumption, the body temperature indicating the metabolic state and thus also the glucose consumption) may provide valuable information for performing a prediction, the amount and type of available data will depend on the day and time when the user performs the prediction: if the user performs the prediction in the morning right after having performed an ECG, current data from all said sensors may be available. If the user performs the same prediction on another day at evening time, no current ECG data and no current data from the fitness bracelet may be available.

By providing an application program that dynamically determines the number and type of available data sources and hence also the amount and type of the available current input data on which a prediction can be based on, and by dynamically identifying and using a particular one of a plurality of prediction logics that is able to compute a prediction based on the particular set of data currently available, a highly flexible diabetes management is provided that is able to provide highly accurate predictions of future physiological states and treatment recommendations, because the prediction is always based on the largest amount of input data currently available. Thus, embodiments of the invention are based on selecting the prediction algorithm dynamically based on the data currently available rather than providing a prediction logic that requires the hardware to provide a defined set of input data and thus implicitly limits the data a prediction is based on to the data provided by sensors which are surely available.

For example, the patient data can be collected by the client application regularly and can be regularly synchronized with a remote copy of all hitherto collected patient data ("patient history") that is stored remotely and managed by the server application. The synchronization is performed via a network, e.g. the internet. For example, the collection and synchronization of patient data can be performed once every 1-5 minutes.

According to some embodiments, server application, in response to receiving the currently collected patient data from the client application, performs an analysis of the received data and identifies a prediction logic in that is able to predict, using the received data as input, the future physiologic state and/or a treatment suggestion. The treatment suggestion can in particular comprise a recommended basal rate and optionally also a recommended bolus.

The data collection by the client application can be implemented based on a push or pull mechanism. For example, the client application can try to receive newly collected data from all data collection units currently coupled to the mobile computing device at predefined time intervals, e.g. every five minutes. Alternatively, the receiving of new data from one of the data collection units, e.g. a glucose meter, that is triggered by this data collection unit may cause the client application to check if in addition any one of the other data collection units can provide new patient related data.

Said features may be advantageous, because said features may ensure that always the broadest possible databases is used for a prediction without imposing any limitations to the system, because neither the presence of e.g. a temperature sensor nor of a gyroscope etc. may be necessary for performing the prediction. Hence, the user may use the system for performing a client-side and/or server-side prediction at an arbitrary time and at an arbitrary day. The quality of the prediction may be affected by the number and type of the data collection units currently coupled to the portable computing system, but in any case it is ensured that the prediction can be performed.

According to embodiments, the server application is further configured for receiving current environmental data from one or more data broadcast services via the network. The current environmental data selectively comprises environmental parameter values currently measured in a geographic region where the portable computing device is currently located.

For example, the server application can be configured for receiving weather data, in particular temperature and/or humidity data, from a weather broadcast service. The weather data may be specifically received for the village, municipal district, city or other geographic region where the patient is currently located. In some example embodiments, the geographic region has a size of at least 1 km$^2$, preferably of at least 5 km$^2$. In addition, optionally, any other data that is known to correlate with or that is suspected to correlate with a particular physiological parameter that has an impact on the diabetes-related health state of the patient could likewise be requested from a broadcasting service via the network, e.g. current air pollution data of the geographic region, or the current traffic situation in the geographic region, or the like.

In addition, or alternatively, the server application is configured for receiving future environmental data from one or more data broadcast services via the network. The future environmental data selectively comprises environmental parameter values predicted to be measured in the future in a geographic region where the portable computing device is currently located. In addition, optionally, any other data that is known to correlate with or that is suspected to correlate with a particular physiological parameter that has an impact on the diabetes -related health state of the patient could likewise be requested from a broadcasting service via the network, e.g. future, predicted air pollution data of the geographic region, or a future, predicted current traffic situation in the geographic region, or the like.

The automated identification of the one out of the plurality of prediction logics is further performed as a function of the type of the received future or current environmental data.

For example, the data broadcast services may comprise a weather broadcast service, a traffic broadcasting service, an air pollution broadcasting service, or the like.

Using region-specific data provided by data broadcast services may be advantageous as the amount of data that is exchanged between the server application and the broadcasting service is reduced and it is ensured that the received data is or could possibly be of physiological relevance for the patient.

According to embodiments, the environmental data comprises a geographic-region-temperature measured or predicted to be measurable in the geographic region. In some example embodiments, the geographic region has a size of at least 1 km$^2$, preferably of at least 5 km$^2$.

According to some embodiments, the data collected by the one of the data sources coupled to the portable computing device is a patient-proximal temperature. The patient-proximal temperature is the body temperature of the patient or is the ambient temperature of the air surrounding the patient in a distance of at least 1 cm.

In addition, or alternatively, the one identified prediction logic is configured to compute a temperature-derived stress score as a function of at least the patient-proximal temperature and the geographic-region-temperature. The temperature-derived stress score is indicative of the physiological and optional also psychological stress level of the patient.

In some embodiments, the one identified prediction logic is configured to remotely compute the prediction as a function of at least the current patient data, the history patient data of the patient and of the temperature-derived stress score.

Using, in addition to the other patient data, both a patient-proximal temperature, e.g. the body temperature, and the geographic-region temperature for performing the prediction has been observed to provide significantly better prediction results compared with using a body temperature or a region-specific temperature alone. Without the intention to be bound by any theory, applicant believes that the patient-proximal temperature is often very different from the geographic-region temperature, and this differences induces stress that results in an increased requirement for insulin. Typically, the outside-temperature is significantly below 37°C and even below the typical room temperature of about 20°C within buildings. In some countries, it is also possible that the outside temperature is much higher than the room temperature generated by air conditioning within buildings.

A body temperature of about 38°C may - independent of the environmental temperature - indicate increased metabolic activity or even an infection with a pathogen and may indicate an increased requirement for insulin. Thus, in some cases, a body temperature alone may indicate a situation that is physiologically and/or psychologically stressful.

In other use case scenarios, applicant has observed that only a combination of the body temperature and the environmental temperature will reveal a "stressful" situation. For example, the body temperature measured at the skin surface of an arm may be 32°C. This may be the "normal" temperature at the skin surface at this region in case the "inner" body temperature of the patient is 37°C and the temperature of the environmental air is about 20°C. Thus, this body temperature alone is not indicative of an increased stress level and correspondingly increased insulin requirement. Likewise, if an ambient temperature of 20°C is obtained as the "patient-proximal-temperature", this temperature is not indicative of increased stress, because 20°C is the temperature preferred by most people. However, if the geographic-region temperature is only about 5°C or is about 40°C, there is a strong temperature difference between the outside temperature and the patient proximal temperature, and strong temperature differences when leaving or entering buildings can cause considerable physiological and psychological stress and influence a person's metabolism, including the glucose metabolism. Hence, strong temperature differences may induce stress in the patient and may increase the amount of insulin needed. In case the temperature differences predicted for a future moment in time, the prediction may return that an increased amount of insulin is required in the future and the treatment recommendation generated by the prediction may be adapted accordingly.

According to preferred embodiments, the prediction algorithm is a machine learning algorithm having been trained on patient history being indicative of the physiologic reaction of a particular patient in respect to a large temperature differences between the patient-proximal temperature and the geographic-region temperature obtained for a particular person. Using a machine learning algorithm having been trained on patient-specific, historic data which correlates temperature differences with the physiologic state (blood glucose level, blood pressure, amount of insulin that was injected to correct the glucose level, etc.) may have the advantage of an increased accuracy of the prediction, because the reaction of a patient to temperature differences has been observed to be highly patient-specific. In a further beneficial aspect, the predicted treatment recommendations can include geographic information, e.g. a recommendation not to visit certain places during certain days or times of day. In addition, or alternatively, the predicted treatment recommendation can comprise a recommendation to increase the insulin dose (base level or bolus) in case the patient should visit a certain place at a certain days or time of days for which particularly high or low temperatures have been predicted.

The client application comprises at least one prediction logic configured for computing a prediction of a future physiologic state of the patient.

Although medical products tend to be tested exhaustively, there does not exist a 100% certainty that defective needles or other consumables used for injecting insulin are identified in the production process. In case the deficiency does not have a tremendous effect on the insulin dose that is applied to through the needle, the deficiency of the needles of a particular product batch may not be detected at all. However, if the physiological data of hundreds or even thousands of patients using needles of a particular product batch are statistically evaluated and compared with the physiological data of other patients using needles of other product batches, it is possible to detect also minor deficiencies, e.g. an only slightly decreased inner needle diameter that slightly but systematically reduces the amount of insulin applied through the needle.

In addition, or alternatively, the type and/or lot number of other consumables can be sensed by one of the data collection units and can be forwarded, via the client application, to the server application. The server application then performs a statistical analysis of the type or lot number of the other consumables and of physiologic parameter values obtained for a plurality of different patients. Examples for these consumables could be the plastic duct used for transporting insulin from a reservoir of the insulin pump to the needle, the insulin reservoir, and the like.

Thus, the collection of a needle type ID or a needle lot number by one of the data collection units, the server-side statistical analysis of physiologic patient data and needle-related data of a plurality of patients, and the sending of a command and or a warning message from the server application program to the client application programs of all patients using a defective needle, may have the advantage that defective needles or other defective consumables can be identified even in case the effect of this defect is small. Thus, patient safety is increased, because the detection of defective medical consumables used for treating diabetes is accelerated and made faster and more sensitive.

As mentioned above, the patient data as well as data on the type and lot number of any consumables used for injecting insulin or for measuring physiological parameters in a patient may be collected regularly and may be synchronized regularly, e.g. every 1-5 minutes, with the server application via the network. The server application, in response to receiving the data, respectively performs an analysis of the received data and identifies a prediction logic in that is able to predict, based on the data received as input, the future physiologic state and/or a treatment suggestion, in particular a recommended basal rate and optionally also a recommended bolus.

According to embodiments, the history of the patient data used by the server application for the prediction is a remote copy of a local history of the patient's patient data. The local history is stored on the portable computing device. The remote history is stored on a storage medium accessible by the server computer. The client application and the server application are respectively configured to repeatedly and fully automatically interoperate with each other such that changes in the local history of the patient's patient data are propagated to the remote history.

The synchronization of patient data may be advantageous, because the client application is enabled to perform the prediction even in case a network connection to the server application is not available. This provides more freedom to the patient, because the patient can receive treatment recommendations and prediction results from the client application also in geographic regions or buildings where an Internet connection is not available. Furthermore, as no network traffic is generated, the prediction results are typically obtained faster if the prediction is performed locally on the portable computing device. According to preferred embodiments, the set of dynamically selectable prediction logics that is stored at the client side (the portable computing device) is identical or very similar (e.g. a large subset of) to the set of dynamically selectable prediction logics that are stored at the server side and used by the server application program. However, the server application program may comprise additional prediction logics which take into account also statistical analysis data derived from the data of many other patients, e.g. of patients located in the same geographic region like the patient for whom the prediction is to be computed.

Thus, in case there is a network connection available, the prediction is delegated to and performed by the server application instead of the client application. This may be advantageous, in particular in case the portable computing device is a smart watch or other wearable computer having only very limited computational power, because CPU and memory resources of the portable computing device are saved. Moreover, the prediction result of a server-side prediction is typically more accurate, because the server application at least in some embodiments is adapted to take statistical data of other patients into account when performing the prediction. Furthermore, the prediction performed by the server can comprise warnings in respect to defective needles and other consumables. These warnings are also obtained based on a statistical analysis of the data of many different patients.

According to embodiments, the client application implements local copies of at least some of the plurality of prediction logics. The client application is configured for locally computing the prediction of the future physiologic state of the patient. In addition, or alternatively, the client application is configured for computing the treatment suggestion for the patient. The client application is further configured for receiving current patient data from the first and other data collection units currently being operatively coupled to the client application and for automatically identifying one out of the plurality of local copies of the prediction logics as a function of the number and type of the currently coupled data collection units. The one identified prediction logic is adapted to use the totality of the received patient data as input.

The client application is further configured to apply the identified local prediction logic on at least the current patient data and on a local history of the patient's patient data for computing the prediction; and for outputting the prediction. For example, the client application can display the prediction result in form of a message on a screen of the portable computing device.

In preferred embodiments, the server application is adapted to perform the remote computation of the prediction in response to the request of the client application. In addition, or alternatively, the server application is adapted to perform the remote prediction in response to receiving new patient-related data during a synchronization operation between the client and the server application.

According to the invention, , each of the one or more data collection units is selected from a group comprising:

- a glucose monitoring device; the data collected by the glucose measuring device is a measured blood glucose level of the patient; for example, the glucose monitoring device can be a continuous glucose monitoring (CGM) device;

- a sensor coupled to an insulin pump; the sensor is adapted to receive data from an insulin injection needle of the insulin pump; the data collected by the sensor is an identifier of the type and/or lot number of the insulin injection needle;

- a motion tracking sensor; preferably, the motion tracking sensor is adapted to monitor and automatically determine the transport medium and/or type of movement. For example, the motion tracking sensor can be adapted to automatically determine if a user is walking, running, riding a bike or riding a horse; this information may indicate different levels of activity and hence different levels of insulin amounts required; According to some embodiments, the motion tracking sensor already includes a program logic for automatically detecting the transport medium and/or the type of movement; according to other embodiments, the motion tracking sensor is adapted to interoperate with a remote service, e.g. a cloud service offered by the vendor or manufacturer of the motion tracking sensor and to provide motion raw data to the remote service for enabling the remote service to automatically determine the transport medium and/or type of motion, and to receive the determined transport medium or the determined type of motion from the remote service; for example, the remote tracking sensor can be a fitness bracelet or an integral part thereof; the data provided by the motion tracking sensor is the transport medium and/or type of motion of the user; optionally, the provided data may in addition comprise movement raw data, e.g. the positions of the user at different moments in time;

- an ECG (electrocardiogram) device; the data collected by the ECG device is physiological measurement data, in particular an electrocardiogram and/or data values derived therefrom; for example, the data collected by the ECG device can comprise heart beat and characteristics thereof, e.g. the P wave, PR interval, QRS complex, J-point, ST segment, T wave and other parameters; the data collected by the ECG device is indicative of the physical activity of the patient; the higher the physical activity, the lower the amount of insulin required;

- a respirometer, the data collected by the respirometer being physiological measurement data, in particular a rate of oxygen and/or carbon dioxide exchange; this data is also indicative of the physical activity of the patient; low level of oxygen or high level of $CO_2$ indicate a bad physiological condition and indicate that a lower amount of insulin is required for effectively reducing the blood glucose level;

- a scale, the data collected by the scale being the body weight of the patient; applicant has observed that a high body weight correlates with an increased amount of insulin required;

- a thermometer, the data collected by the thermometer being a patient-proximal temperature; the patient-proximal temperature is the body temperature of the patient or is the temperature of the air surrounding the patient in a distance smaller than 3 meter; typically, the higher the body temperature, the higher the metabolism of the patient and the higher the amount of insulin needed; likewise, the higher the temperature of the air surrounding a patient, the higher the metabolism and the higher the amount of insulin needed; preferably, the patient-proximal temperature is measured in the immediate vicinity of the patient, e.g. < 3 m, more preferentially < 2 m;

- an insulin pump logging unit; the data collected by the insulin pump logging unit are the time and/or amount of insulin applied to the patient; the insulin can be applied manually, semi-automatically or fully automatically;

Preferably, the portable computing device is operatively coupled to a plurality of the above mentioned data collection units. This may be advantageous as a broad data basis is provided for the prediction and the prediction accuracy may be increased.

According to embodiments, the client application is configured for displaying the locally or remotely computed prediction on a display of the portable computing device. The displayed prediction comprises an amount of insulin that corresponds to a basal insulin rate predicted to be advisable for the patient. In addition, or alternatively, the client application is configured for sending a command to an insulin pump of the patient, the command causing the insulin pump to inject an amount of insulin to the patient that corresponds to a basal insulin rate specified in the prediction.

Displaying the prediction on a screen may be advantageous as the patient is enabled to inspect the prediction result and check whether or not he or she wants to follow the computed recommendations or not. Automatically sending a command to the insulin pump hay increase the convenience of use for the patient as the insulin basal rate is automatically adapted to modified physiological and environmental parameters without requiring the patient to be actively involved in the process.

According to embodiments, the client application and/or the server application is further configured for: accessing data on the amount of consumables for insulin injection currently available to the patient; predicting the amount of consumables required by the patient in a future time interval as a function of at least the current patient data and the patient history; in response to determining that the amount of available consumables is below the amount of predicted, required consumables, automatically ordering new consumables from one or more suppliers or sending an invitation message to a health care provider to prescribe the new consumables.

Whether or not the consumables can automatically be ordered by the client application or an electronic ordering system connected to the client application, or whether the patient requires a prescription of the health care provider before he or she can place the order may depend on the type of consumable and the jurisdiction in the respective country. According to embodiments, the client application can be configured by the end user (the patient) and/or by the vendor or developer of the client application such that the program routines implementing the ordering of new consumables comply to the legal requirements of the respective country.

In a further aspect, the invention relates to a portable computing device assigned to a patient with diabetes. The portable computing device comprises a client application, one or more interfaces for reversibly coupling the client application operatively to a first and other data collection units, and a network interface. Each data collection unit is configured to collect data of a respective different data type and the data are related to the patient; The network interface is configured to operatively couple the client application to a server application running on a remote server. The client application is configured for locally computing a prediction of a future physiologic state of the patient and/or for locally computing a prediction of a treatment suggestion for the patient. The client application implements and maintains a plurality of different prediction logics configured to compute a prediction of a future physiologic state of the patient and/or a treatment suggestion for the patient.

The client application is configured for:

- receiving a largest amount of first and other currently available patient data from the data collection units currently being operatively coupled to the client application;

  ◦ the first data being received from the first data collection unit, the first data collection unit comprising a glucose meter currently coupled to the portable computing de-vice,

  ◦ wherein the client application is further configured for, upon receiving the first data, trying to receive the other data from the other data collection units currently coupled to the portable computing device,

- identifying the one or more data collection units having collected the current patient data,

- dynamically determining a number and type of the identified one or more data collection units,

- automatically identifying and selecting a particular one out of the plurality of the prediction logics as a function of the number and type of the currently coupled data collection units, that is able to compute a prediction based on the largest amount of data currently available, the one identified prediction logic being adapted to use the totality of the received patient data as input;

- applying the identified local prediction logic on at least the current patient data and on a local history of the patient's patient data for computing the prediction; and

- outputting the prediction. For example, the predicted physiologic state of the patient and/or the predicted treatment suggestion for the patient can be displayed on a screen of the portable computing device, thereby allowing the patient to read the prediction and take appropriate actions. For example, the patient may adapt the insulin basal dose, change the insulin injection needle, call an emergency service or block the client application from automatically calling an emergency service.

- sending a command to an insulin pump of the patient, the command causing the insulin pump to inject an amount of insulin to the patient that corresponds to a basal insulin rate specified in the prediction,

- wherein each of the other data collection units is selected from a group comprising:

  ◦ a motion tracking sensor;

  ◦ an ECG (electrocardiogram) device, the data collected by the ECG device being physiological measurement data, in particular an electrocardiogram and/or data values derived therefrom;

  ◦ a respirometer, the data collected by the respirometer being physiological measurement data, in particular a rate of oxygen and/or carbon dioxide exchange;

∘ a scale, the data collected by the scale being the body weight of the patient;

∘ a thermometer, the data collected by the thermometer being a patient-proximal temperature, the patient-proximal temperature being the body temperature of the patient or being the temperature of the air surrounding the patient;

∘ an insulin pump logging unit, the data collected by the insulin pump logging unit being the time and/or amount of insulin applied to the patient.

According to embodiments, the portable computing device is a smart watch. This may be beneficial as smart watches are particularly convenient to wear, have a low weight and are inconspicuous, so a smart watch worn by a particular person will not identify the person as diabetes patient.

A "**diabetes management software**" as used herein is a data processing system comprising one or more software programs that help persons with diabetes (e.g. Type 1, Type 2 or other forms of diabetes) to manage the data associated with their disease. The data can in particular comprise blood test results from a glucose meter, diabetes logbooks, basal and bolus settings for an insulin pump, records of actual insulin delivery, records of carbohydrates eaten and boluses, statistical evaluations, manual log entries for exercise and other factors, etc.

A "**smart watch**" as used herein is a wearable computer in the form of a wristwatch. In particular, the wearable computer can comprise one or more processor and a storage medium comprising one or more mobile apps and a mobile operating system. In addition, the smart watch may comprise a near filed communication interface, e.g. a Bluetooth or WiFi connectivity. Some models, called 'watch phones' (or vice versa), have complete functionality of a typical smartphone. A smart watch has an electronic visual display, e.g. an LCD or OLED or Hologram or use transflective or electronic paper, to consume less power. The smart watch can have a rechargeable battery and can have a touchscreen. In some embodiments, the smart watch comprises or can be reversibly coupled with sensor devices like digital cameras, thermometers, accelerometers, pedometers, heart rate monitors, altimeters, barometers, compasses, GPS receivers, etc.

A "**data collection unit**" as used herein is a piece of software-, firmware and/or hardware based logic that is adapted to collect data and to forward the collected data to a receiving entity, e.g. a portable computing device of a patient. The collected data can be forwarded in its original format or can be forwarded after some processing steps, e.g. after transforming the data into a predefined data format that is interpretable by the receiving entity. A data collection unit can be, for example, a software-based program module adapted to access events in an electronic calendar of a patient and adapted to compute a stress level from the event data. Likewise, a data collection unit can be a sensor device, e.g. a thermometer, a chemical measurement device like a glucose meter, a camera or the like. A data collection unit can be any device, module, or subsystem whose purpose is to detect events or changes in its environment and send the information to a receiving entity, e.g. other electronics, frequently a computer processor.

A "**geographic-region-temperature**" as used herein is a temperature that is measured or predicted to be measurable in a particular geographic region. For example, the geographic region can be the city or city district or village where a patient currently resides or is predicted to reside at a particular time in the future. Preferably, the geographic-region-temperature is a temperature that is measured outside of the immediate environment of a patient and is basically not influenced by the presence of the patient. Hence, the geographic-region-temperature is not a body temperature of a patient. For example, the geographic-region-temperature can be provided by a weather-broadcast service that receives measurement data, e.g. temperature and moisture, from many weather stations in a district. In particular, the geographic-region-temperature can be an outdoor temperature.

To the contrary, a "**patient-proximal-temperature**" is a body temperature of the patient or is an ambient temperature of the patient.

An "**ambient temperature**" as used herein is a temperature measured in the immediate vicinity of a patient, but measured with a minimum distance of at least 1 cm from the skin surface of the patient. For example, the ambient temperature can the temperature of the air surrounding the patient measured in a distance of at least 1 cm from the skin. For example, the ambient temperature can be the room temperature of a room where a patient is currently located or is predicted to be located at a particular time in the future. In particular, the ambient temperature can be the temperature of the air, within a room or within a building.

The "**body temperature**" of a patient is the temperature that is measured by a thermometer that is placed inside of the human body or that is placed on the skin surface of the human body. A person's normal body temperature is 98.6 degrees Fahrenheit or 37 degrees Celsius. The body temperature can be measured by a thermometer that is placed on the surface of the skin surface of an arm or leg or that is placed under an arm of the patient. The term "body temperature" as used herein is not limited to the temperature of the blood or inner regions of the body. Although the temperature on the surface of a patient's arm or leg may be several degrees Centigrade below the body temperature that would be obtained by a thermometer placed inside a patient's body or below an arm, the body temperature at the skin surface of the arm or leg still correlates with and is indicative of the "inner" body temperature of the patient and will herein be likewise be considered as "body temperature". Hence, the body temperature is a physiologic parameter of the patient and is indicative of other physiologic parameters, e.g. metabolic state and insulin requirement.

A "**stress score**" as used herein is a data value, in particular a numerical data value, that is indicative of the physiologic stress of a patient. The physiologic stress may be caused by one or more different factors such as temperature-differences, workplace stress induced by many appointments, and may also be indicative of psychological stress.

A "**temperature-derived stress score**" as used herein is a stress score that is computed as a function of at least the patient-proximal temperature and the geographic-region-temperature. The temperature-derived stress score is indicative of the physiological stress level of the patient.

A "**calendar-derived stress score**" as used herein is a stress score that is computed as a function of number and/or type of events in an electronic calendar of a patient. Preferably, the calendar-derived stress score is computed such that the higher the number of appointments and the higher the economic relevance of the appointments for the patient, the higher the calendar-derived stress score.

A "**currently active insulin**", "bolus on board" or "insulin on board" as used herein is the number of units of rapid insulin which are still working (available in a body) at a particular time. This measure helps in deciding whether more insulin or more carbohydrate is needed. A low blood glucose level alone should not be used as an indicator that more insulin is needed. Rather, in addition to the current blood glucose level, the currently active insulin level should be determined in order to determine how much bolus is on board before taking more insulin.

The "**currently active carbohydrates**" indicates the amount of carbohydrates , in particular glucose, contained in the blood.

## Brief description of the drawings

In the following embodiments of the invention are explained in greater detail, by way of example only, making reference to the drawings in which:

Figure 1    is a block diagram of a distributed diabetes management system.

Figure 2    is a flowchart of a method of deciding if the prediction should be executed locally or remotely.

Figure 3A   is a flowchart of a local (client-side) prediction.

Figure 3B   is a flowchart of a remote (server-side) prediction.

Figure 4    is a schematic drawing of a smart watch display.

Figure 5    is a block diagram of an insulin pump comprising an injection needle.

Figure 6    shows different embodiments of an insulin injection needle.

Figure 7    depicts two systems comprising an insulin pump and a needle.

Figure 8    is a system with a mobile device comprising a needle-data-sensor.

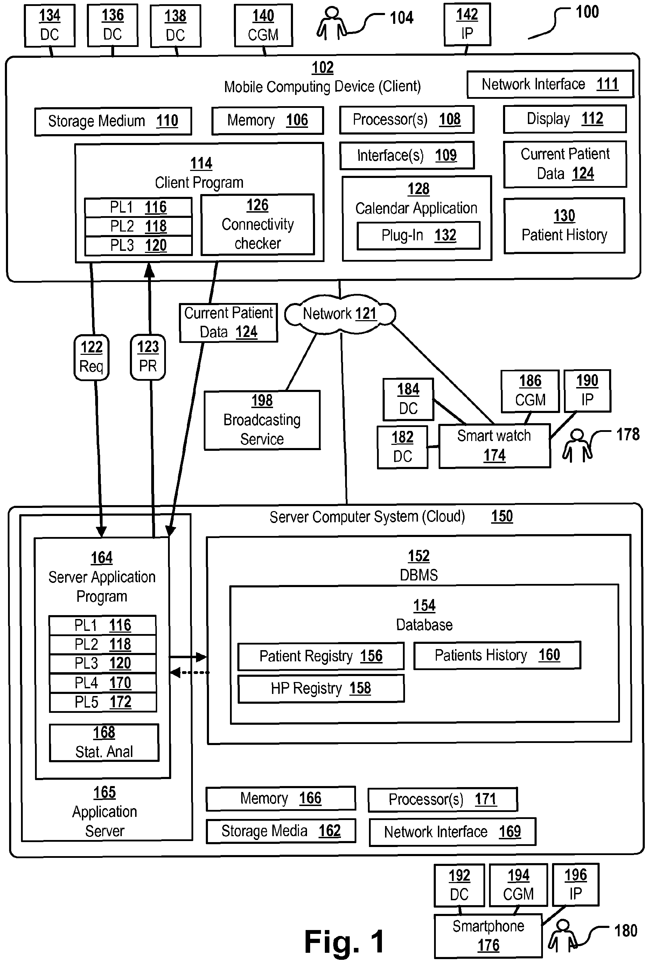

**Figure 1** is a block diagram of a distributed diabetes management system 100. The system comprises a server computer 150 and one or more battery powered computing devices. For example, the server computer 150 can be implemented as a standard computer comprising one or more processors, a main memory and one or more non-volatile storage media. The server computer can likewise be implemented as a distributed computer system, e.g. a cloud computer system, wherein a plurality of resources (CPUs, main memory, non-volatile storage media, in particular hard disk drives) are connected with each other via a network. A cloud computing system enables ubiquitous access to shared pools of configurable system resources and higher-level services that can be rapidly provisioned with minimal management effort, often over the Internet. Cloud computing relies on sharing of resources to achieve coherence and economies of scale, similar to a public utility. The server computer system 150 comprises a main memory 166, one or more non-volatile storage media 162, one or more processors 171 and a network interface 169 for connecting the server computer with one or more portable computing devices 102, 174, 176 and, optionally, with one or more broadcasting services 198 via a network 121. Examples for the non-volatile storage media 162 are a magnetic tape, an optical disc or an electronic data storage which requires electrical power to store and retrieve data. A storage medium is a physical material that holds information. Digital information can be stored on electronic media in many different recording formats. The storage medium 162 can bin particular be a hard disc drive. A hard disk drive (HDD), is a data storage device that uses magnetic storage to store and retrieve digital information using one or more rigid rapidly rotating disks (platters) coated with magnetic material. The platters are paired with magnetic heads, usually arranged on a moving actuator arm, which read and write data to the platter surfaces. HDDs are a type of non-volatile storage, retaining stored data even when powered off. Flash memory has a growing share of the market for hard disc drives and secondary storage, in the form of solid-state drives (SSDs).

The battery powered portable computing device can be, for example, a smart phone 102, 176, a smart watch 174, a tablet computer or the like. Each portable computing device is specifically assigned to a user who is a diabetes patient trying to monitor and control his or her disease with the help of the respectively assigned portable computing device.

For example, computing device 102 is specifically assigned to patient 104. The portable computing device 102 comprises a non-volatile storage medium 110, e.g. a hard disk drive, a main memory 106, one or more processors 108, a display 112, e.g. a touchscreen, and a network interface 111 for communicatively coupling the mobile computing device via the network 121 to the server computer 150. In addition, the mobile computing device comprises one or more interfaces 109 for coupling the computing device 102 was one or more data collection units 134, 136, 138, 140, 142. For example, the interface 109 can be a Bluetooth interface, a WiFi interface or any other type of interface for exchanging data, in particular a near field communication interface. In some embodiments, one or more of the data collection units can also be an integral part of the mobile communication device. In this case, the data exchange with the client program is not performed via the interface 109 but rather via other interfaces which can be part of the operating system of the portable computing device.

Each portable computing device 102, 174, 176 comprises a client application program 114 which is adapted to interoperate with a server application program 164 hosted by the server computer 150. Therefore, the portable computing devices 102, 174, 176 are also referred to as "client devices".

The client application 114 comprises a plurality of prediction logics 116, 118, 120 which are respectively adapted for computing a prediction of a future physiologic state of the patient and/or for computing a treatment suggestion for the patient for the near and far future. For example, a first prediction logic. Prediction logic 116 could be adapted to predict a future blood glucose level of a patient using the currently measured blood glucose level and the current body temperature of the patient as input. Prediction logic 118 could be adapted to predict a future blood glucose level of the patient using the currently measured blood glucose level and the currently measured calendar-derived stress level of the patient as input. Prediction logic 120 could be adapted to predict a future blood glucose level of the patient using the currently measured blood glucose level, the currently measured calendar-derived stress level of the patient, the currently measured body temperature of the patient and an activity score of the patient derived from a GPS based movement pattern over the last two days as input.

The server application 164 comprises a duplication of the prediction logics comprised by the client application. According to preferred embodiments, the server application comprises additional prediction logics 170, 172 adapted to use input data that is not available to the client application in addition to the current patients data 124 received from the client application for performing the prediction. For example, the server application program 164 can be adapted to request additional data from broadcasting services 198, e.g. a weather forecast service, via the network 121. In addition, or alternatively, the server application program 164 can be adapted to use the statistical information derived from a plurality of patients 160 having registered at the server computer 150 as additional input data for performing the prediction.

Hence, the plurality of prediction logics managed by the server application 164 are configured to predict the same type of patient related information, i.e., a future physiologic state or a recommended treatment option, but comprise prediction logics which are capable of performing this predictions based on a broader data basis than the client application.

The portable computing device 102 is operatively coupled to a plurality of data collection units 134, 136, 140, 132, 142. The coupling is reversible. For example, the coupling can comprise establishing a Bluetooth or WiFi connection between a data source and the portable computing device 102 first, and then establishing a communication link between the client application 114 and some software or hardware interface contained in the coupled data collection units using the established Bluetooth or WiFi connection. Likewise, portable computing device 174 assigned to patient 178 is reversibly coupled to data collection units 182, 184, 186, 190. Portable computing device 176 assigned to patient 180 is reversibly coupled to data collection units 192, 1194, 196. Some of the data collection units can also be an integral part of a portable computing device. For example, in case the portable computing device 102 comprises a GPS sensor or a gyrometer, the GPS sensor or the gyroscope can be used by the client application program as data collection units.

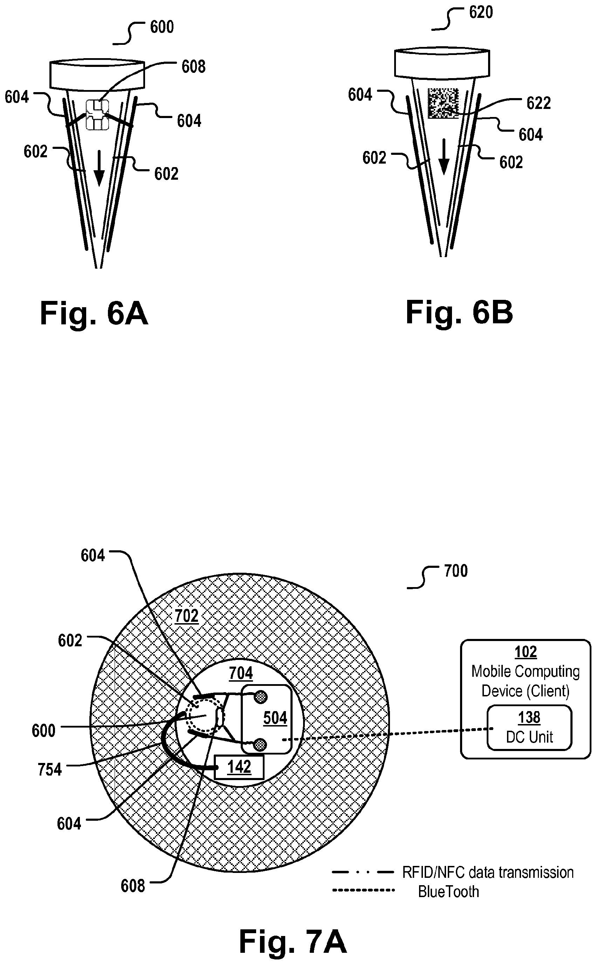

Device-internal data collection units may be permanently coupled to the client application system, e.g. because they may continuously provide measurement data to the client application directly or via the operating system of the portable communication device. Device-internal data collection units may also be reversibly coupled to the client application, e.g. because the user may be allowed to activate or deactivate the respective functionality, e.g. the GPS sensor functionality. The number and type of data collection units coupled to a respective portable computing device can be very heterogeneous and variable. For example, the data collection units 134, 136, 138, 182, 184, 192 and 194 can comprise thermometers, scales, gyrometer, an electronic ECG sensor, a spirometer, a fitness bracelet comprising one or more of these data collection unit, and others. Preferably, one 140, 186, 194 of the data collection units currently coupled to the client device is blood glucose meter, in particular a continuous glucose meter (CGM). Optionally, a patient 104, 178, 180 uses an insulin pump for injecting a recommended basal rate of insulin. According to preferred embodiments, the insulin pump comprises at least one sensor adapted to collect data related to the injection of insulin by the insulin pump, e.g. type, product charge, or age of the consumables used for the insulin injection. For example, the consumables can comprise an injection needle as depicted, for example, in figure 6. In addition, or alternatively, the consumables can comprise a plastic duct connecting an insulin reservoir with the injection needle and/or the insulin reservoir having been inserted into the pump. This sensor is preferably also operatively coupled to the client application and is also used as a data collection unit. The sensing of the type of consumables used by a patient may allow predicting, by the client application program or by the server application program, a time when the patient will run out of the respective type of consumables. In addition, the sensing of the type of consumables used by many patients in combination with the acquiring of physiologic parameter values from many patients may allow automatically determining, by the server application having access to the data of a plurality of patients, to determine if a particular product batch is defective and causes small but measurable and statistically significant undesired effects on the health of many patients.

Each data collection unit is configured to collect data of a respective data type related to the patient and forwarded to the client application 114. The data collection and forwarding of the data to the client application can be performed synchronously or asynchronously. Asynchronous data collection means that different data collection units can be configured to collect data at different times and after different time intervals. Synchronous data collection means that all or at least some data collection units perform the data collection at the same time and after identical time intervals. Likewise, the forwarding of the collected data from the respective data collection units to the client application program can be performed synchronously or asynchronously, depending on the embodiment.

For example, a GPS sensor can be configured to determine its current position (and hence, the current position of the patient) every five minutes and to immediately forward the collected position to the client application. The client application is enabled to generate a movement profile that is indicative of the physical activity of the patient. Furthermore, this information can be used for extrapolating and predicting future movements and positions of the patient, thereby predicting a future position or future activity state of the patient. However, as the GPS sensor may merely allow determine a movement based on a change in positional data over time, preferably a movement tracking sensor is used in addition to or instead of the GPS sensor, whereby the movement tracking sensor is further adapted to determine the type of movement or the transport medium used for performing a movement.

Another data collection unit, e.g. a CGM, can be configured to detect the current blood glucose level of a patient every three minutes and to immediately forward the measured blood glucose level to the client application.

A still further data collection unit, e.g. an ECG machine, may be coupled to the client application by the patient only once in a day, e.g. only in the morning at 8 AM or at changing times once a day, or even irregularly every few days. The collected ECG data is forwarded to the client application. Even in case the patient may not use this ECG machine regularly, the ECG data, if available, may help increasing the accuracy, and as the prediction algorithm is selected dynamically in dependence on the currently available data, the patient may benefit from the ECG data even in case the ECG data is not obtained regularly.

The client application is configured to store all data received from any of the data collection units in the storage medium 110 in the form of current patient data 124. Patient data received from the data collection units that has already been used for performing a prediction or patients data that exceeds a threshold for maximum age is stored on the storage medium 110 as "patient history" 130. Preferably, the data collected for many different data collection units is transformed into a unified, well-defined format, e.g. an XML format, before it is stored as current patient data 124 and later as patient history data 130. In some embodiments, the current patient data and/or the patient history data is stored in a relational database, e.g. MySQL or PostgreSQL or SAP HANA database.

In some embodiments, the receiving of a current blood glucose level from a CGM is a trigger event that causes the client program to collect the most recent data values provided by any of the currently coupled to data collection units and use this collected data as the "current patient data" 124. Optionally, also the most recent data values provided by any data collection units that has once been coupled to the client application program but that is currently not coupled is also used as the "current patient data" 124. The current patient data 124 is used by the client program 114 or by the server application program 164 in order to automatically identify the one out of a plurality of available prediction logics which is able to make use of the totality of available current patient data 124 for performing a prediction of a future physiologic state of a patient and/or for computing a treatment recommendation for the patient. The dynamic identification of this particular prediction logic may be beneficial, because the broadest possible databases is used for the prediction, thereby increasing the accuracy of the prediction. Furthermore, the dynamic identification of the prediction logic provides for a highly flexible system that can cope with the fact that the number and type of data collection units that are currently coupled to the portable computing device of the patient may vary strongly from day to day or even within a single day.

The data that is collected by the data collection units coupled to the client device is replicated to the server application 164 running on the server computer 150. In some embodiments, the data replication is performed by sending the currently collected patient data 124 together with a request 122 for performing a prediction regarding the future physiologic state or regarding a recommended treatment option from the client program to the server application program. In other embodiments, the data replication can be performed separate from and independent of the sending of the prediction request 122.

The server computer system comprises a database 154, e.g. a relational database managed by a DBMS 152. The database comprises a registry 156 comprising a plurality of patient accounts and a further registry 158 comprising a plurality of healthcare provider accounts. In addition the totality of current patient data that was replicated from the client device is assigned to the plurality of registered patients is stored in the form of a patient history 160 of the registered patients in the database. Hence, the patient history 130 is a "local" copy of the patient history of patient 104 while the patient history 160 comprises a "remote" copy of the patient history of patient 104, but also a "remote" copy of the patient history of other patients 178, 180.

*Computation of a calendar-derived stress score*

Optionally, each client device can comprise a calendar application 128, e.g. Outlook or a calendar app that is used by the patient to whom the client device is assigned for managing his or her appointments, e.g. meetings, customer talks etc. Preferably, the calendar application comprises a plug-in or add-in 132 which is interoperable with the client program and which implements a calendar-analysis program logic ("CA program logic"). The CA program logic is configured to access and analyze the events stored in the electronic calendar of the calendar application 128 and to compute a calendar-derived stress score. The calendar-derived stress score is computed as a function of the number and type of events which are stored in the electronic calendar and which are identified in the analysis. The calendar-derived stress score is a data value, preferably a numerical value within a defined value range, which is indicative of the physiologic and optionally also psychological stress level of the patient. In general, the more events related to appointments found in the electronic calendar, the higher the calendar-derived stress score.

Optionally,, the CA program logic is an integral part of the client program 114. Alternatively,, the calendar application 128 is not a separate application, but rather a sub-module or sub-function of the client program 114.

For example, , the CA program logic is a machine learning logic, e.g. a neuronal network or a support vector machine that was trained on a patient history 130 and a history of appointments stored in the calendar application 128. The patient history 130 can comprise a plurality of physiological parameters such as blood glucose value, body temperature, active insulin, active carbohydrates, insulin basal rate etc. as well as other parameter values, e.g. calendar-derived stress scores of the patient having been computed in the past. During the training, the CA logic 132 learns how the patient reacts to appointments of different types. For example, the CA logic 132 learns if and how much a particular type of event, e.g. a customer talk, results in a modified pulse, body temperature, skin moisture, insulin consumption and/or blood glucose level. In other words, the machine learning logic learns from correlations of physiological parameters and events and the electronic calendar that have been measured and stored in the past to compute a calendar-derived stress score also for future events and the future time intervals.

For example, the CA logic enables a user to tag each event in his or her electronic calendar that has happened in the past with one of a predefined list of stress level scores. Then, a machine learning algorithm is trained on the tagged events in the calendar, thereby learning to automatically identify and predict the stress level that will be imposed on the patient by future events in the electronic calendar. The calendar-derived stress score can be used as a part of the current patient data 124 collected by one or more data collection units that is forwarded to the server application program 164. Thereby, the server application program is enabled to identify one or more prediction logics which are able to use the calendar-derived stress score, among other patient related data, as input for performing the prediction.

The client program 114 comprises one or more prediction logics respectively configured for computing a prediction of a future physiologic state of the patient and/or for computing a treatment suggestion for the patient as a function also of the calendar-derived stress score (in addition to the other input data described above).

*Computation of a temperature-derived stress scores*

According to some embodiments, the server application 164 is adapted to receive additional input data that is not available to the client application program 114. For example, the server application program can receive current environmental data from one or more data broadcast services 198 via the network. For example, the data broadcast services can be weather forecast services and the current environmental data can comprise environmental parameter values currently measured in a geographic region where the portable computing device is currently located. In addition, or alternatively, the server application program can request the environmental parameter values of a particular geographic region for a future moment in time, e.g. for one or more subsequent days. For example, in case the client application program predicts, based on events data in the electronic calendar of the patient, that the patient will visit a client in a particular city tomorrow, that prediction regarding the future place of the patient can be forwarded to the server application program and used by the server application program to request weather data for the next day from the weather forecast service 198. The request that's, current or future weather data or other form of environmental data is received by the server application via the network. Then, a prediction logic is identified which is able to use, in addition to all other current patient data 124, the received environmental data as input for performing the prediction or for computing the treatment recommendation.

For example, the environmental data can comprise a geographic-region-temperature predicted for a particular town that the patient is predicted to visit tomorrow. One of the data collection units can be a thermometer that is adapted to measure the body temperature of the patient and use the body temperature as the patient-proximal temperature. The current patient data 124 comprises the body temperature and the one identified prediction logic is configured to compute a temperature-derived stress score as a function of the patient-proximal temperature and the geographic-region-temperature. The temperature-derived stress score is indicative of the physiologic and optionally also psychological stress level of the patient. This or a further identified prediction logic is configured to remotely compute the prediction as a function of at least the current patient data 124, the history patient data 160 of the patient and of the temperature-derived stress score.

For example, the temperature-derived stress score TDSC can be computed as follows:

$$TDSC = \frac{|Tgr - Tpp|}{NF}$$

wherein Tgr is the geographic-region-temperature, Tpp is the patient-proximal-temperature, and NF is a normalization factor that is preferably between 20 and 60. For example, in case the Tpp is an ambient temperature of 20°C, Tgr is 45°C and NF is 30, then TDSC is computed as

$$TDSC = \frac{|25|}{30} = 0.83.$$

In an example, the server application program computes a total stress score from the temperature-derived stress score and the calendar-derived stress-score. In this case, the one identified prediction logic is configured to remotely compute the prediction and/or the treatment recommendation as a function of at least the current patient data, the history of the patient data of the patient and of the total stress score.

*Trigger events*

The remote (by the server application) or local (by the client application) identification of a particular prediction logic and computing the prediction or treatment suggestion is triggered according to embodiments of the invention by a trigger event. For example, in case the client application determines that the predefined time interval has lapsed, e.g. a time interval of five minutes, then the client application may use all data provided by any one of the data collection units which are currently coupled to the client device 102 and optionally in addition some additional patient related data that is not older than a predefined maximum age as input for performing the prediction. Alternatively, the event of receiving current patient data from one of the data collection units, in particular a CGM 140, can act as a trigger event for performing the prediction. Still alternatively, the client application may be configured to generate a GUI comprising selectable GUI elements whose selection by the user triggers the performing of the prediction. In addition, or alternatively, the instantiation of the client application can trigger the prediction.

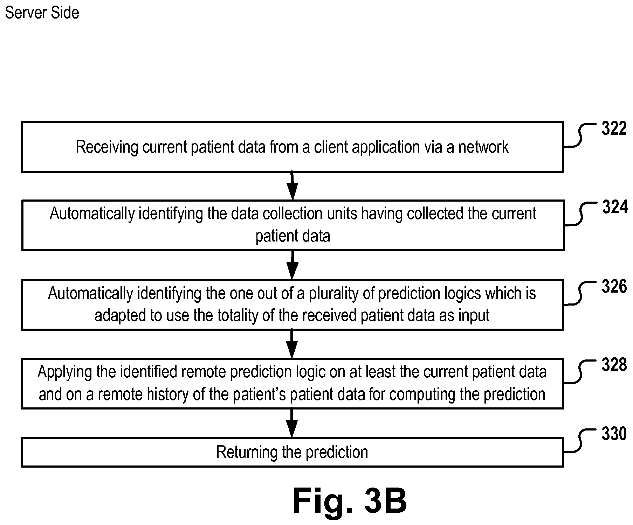

In response to the detection of the trigger event by the client application, a connectivity checker routine 126 of the client program 114 checks, if a network connection to the server application 164 is available. In case the network connection is available, the client application program sends a request 1224 performing a remote computation of the prediction to the server application 164 and receives, in response to the request, a response message 123 form the server application. The response message comprises the result of the prediction. Optionally, the request 122 can comprise the current patient data 124. The remote computation of the prediction is illustrated in detail in figure 3B.

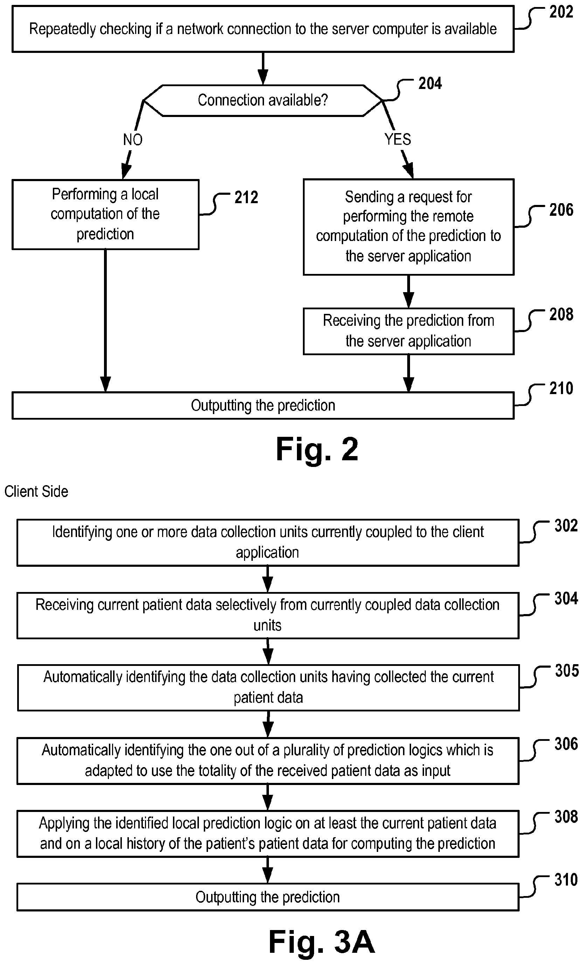

Selectively in case the connection is not available, the client application 114 performs a local computation of the prediction. The local computation of the prediction is illustrated in further detail in figure 3A.

Finally, the client application displays the locally or remotely computed prediction on a display 112, e.g. a touch screen. The prediction comprises an amount of insulin that should be injected to the patient and a future moment in time, according to the prediction. The future moment in time could be the near future, e.g. the next few minutes, or a more distant future, e.g. the next few hours or even days. The future time for which the prediction is performed can be preconfigured in the client application program or server application program and can in some cases even be defined by the individual patient. The recommended insulin amount can have the form of a recommended insulin basal rate or the form of a recommended insulin bolus.

For example, the client application program sends a command to an insulin pump 142 coupled to the client device hosting the client program. The command causes the insulin pump to automatically inject the recommended insulin bolus into the patient. Alternatively, the command modifies the settings of the insulin pump such that the insulin basal rate is set to the predicted recommended insulin basal rate provided by the command. Preferably, the insulin pump comprises an interface that prompts the patient to manually confirm the injection of the modification of the settings of the insulin pump.

**Figure 2** is a flowchart of a method of deciding if the prediction should be executed locally or remotely. The client application 114, 126 can be configured to repeatedly, e.g. every five minutes, check in step 202, 204, if a network connection to the server computer 150 is available. This may not always be the case. For example, in case the patient is currently located in a hospital where the use of smart phones is prohibited or where a network connection via the mobile cellular telecommunication network is not available, the mobile computing device 102 with the client application 114 may not be able to exchange any data that was the server application 164.

Selectively in case the network connection is available, in step 206 the client application sends a request 122 for performing the remote computation of the prediction to the server application 164. The request can comprise current patient data 124 collected by a plurality of data collection units coupled to the portable computing device 102. Optionally, the current patient data 124 can in addition comprise patient data collected by one or more data collection units which are not currently connected, provided the data is not older than a predefined maximum age threshold, e.g. one or two days. The threshold may depend on the type of data that is collected. For example, it can be assumed that the activity profile that is based on a movement profile can be allowed to be one or two days old, while a four-day old body temperature may not reflect a "current" body temperature anymore.

Next in step 208, the client application receives the prediction result from the server application. For example, the prediction result can be received in the form of a response message 123.

In case no network connection is available, the client application in step 212 performs a local computation of the prediction, the disk basically identical to the remote computation, except that the server application programs comprises some additional program routines which are capable of using additional information as input for the prediction first of the additional information can comprise, for example,. data provided by remote broadcasting services 198 and/or data obtained by a statistical analysis 168 performed on the patient history data of the plurality of registered patients.

Next in step 210, the client application outputs the locally or remotely computed prediction result. For example, the client application can display the result on a screen.

**Figure 3A** is a flowchart of a local (client-side) prediction. For example, the method can be executed by a client program 114 is depicted and described for figure 1. The client application program is configured for locally computing the prediction of the future physiologic state of the patient and/or for predicting a treatment suggestion for the patient. The client application program 114 identifies, in a first optional step 302, one or more data collection units which are currently operatively coupled to the client application. In step 304, the client application receives current patient data from the first and other data collection units which are currently operatively coupled to the client application. Steps 302 and 304 can also be implemented as a single step in which the client application receives some data collected by a respective data collection units, thereby implicitly identifying the type of the data collection units that provides the data. Next in step 305, the client application automatically identifies one out of the plurality of prediction logics stored locally on a storage medium accessible to the client application as a function of the number and type of the currently coupled data collection units. The one identified prediction logic must be able to use the totality of the received patient data as input and compute the prediction based on the available input data. Optionally, the input data can comprise additional patient related data that was collected recently, preferably no longer than several hours ago. In this case, the identified prediction logic must be able to use also this additional patient related to data as input for performing the prediction. In some embodiments, the identification is implemented in the form of a program routine that collects the IDs of all data aggregation units having provided some of the current patient data 124 to be used as input and that evaluates a mapping table comprising an assignment of different sets of data-collection-unit-IDs with respective program routines.

In some other embodiments, the patient data received from all coupled data collection units into a single file, e.g. an XML file, wherein the ID or type of the respective data collection units having provided the respective XML file element are tagged with a respective XML element. The identification of the prediction logic is implemented in the form of a program routine that parses the XML file for extracting the patient related data and the IDs or types of the data collection units respectively having provided the data.

In step 308, the client application program applies the one identified local prediction logic on at least the current patient data and on the local history of the patient's data for computing the prediction. Finally, the prediction is outputs in step 310, e.g. by displaying a recommended insulin basis rate on a display screen of the mobile computing device.

**Figure 3B** is a flowchart of a remote (server-side) prediction. For example, the method can be implemented by a server application program 164 as depicted in figure 1. The server application is interoperable with the client application and implements a plurality of prediction logics 116, 118, 120, 170, 172. Each prediction logic is configured for remotely computing a prediction 123 of a future physiologic state of the patient and/or for predicting a treatment suggestion for the patient. The server application receives in step 322 current patient data 124 from the client application via a network 121, e.g. the Internet. The received current patient data comprises data collected by one or more of the data collection units. Next in step 324, the server application program identifies the one or more data collection units having collected the current patient data. Next in step 326, the server application program automatically identifies one out of the plurality of prediction logics as a function of at least the number and type of the identified one or more data collection units. The one identified prediction logic is adapted to use the totality of the received current patient data as input. The identification of the prediction logics can be performed by the server application program as described for the client application program.

Next in step 328, the identified prediction logic is applied on the received current patient data and on a history of the patient's patient data for computing the prediction. Optionally, the current patient data 124 received from the client application program can be supplemented with an additional data, in particular data derived from a statistical evaluation of the patient data of a plurality of patients and/or from broadcasting services.

For example, the data derived from the statistical analysis can indicate that selectively a patient-subgroup using an insulin needle having the same lot number as the insulin needle currently used by the patient have some problems, e.g. show a reduced physiological response to an insulin injection. This could be indicative of a defective insulin needle product batch whose inner diameter is smaller than required, thereby reducing the amount of insulin that is injected into a patient.

According to another example, the data derived from the statistical analysis can indicate that selectively a patient-subgroup currently located in the same city district have an increased insulin requirement. This could be caused by a particular weather condition, or an infectious disease.

According to another example, the data derived from the statistical analysis can indicate that selectively a patient-subgroup using a plastic insulin duct of a particular vendor for longer than three days show a reduced physiological response to an insulin injection. This could be indicative of a clogging of the duct and that the duct needs to be exchanged.

Next in step 330, the prediction is returned to the client application via the network, thereby enabling the client application to display the prediction result on a the screen to the patient. In addition, or alternatively, the prediction result can be forwarded to an insulin pump for automatically reconfiguring the insulin pump, e.g. for increasing or decreasing the insulin basal rate in dependence on the prediction.

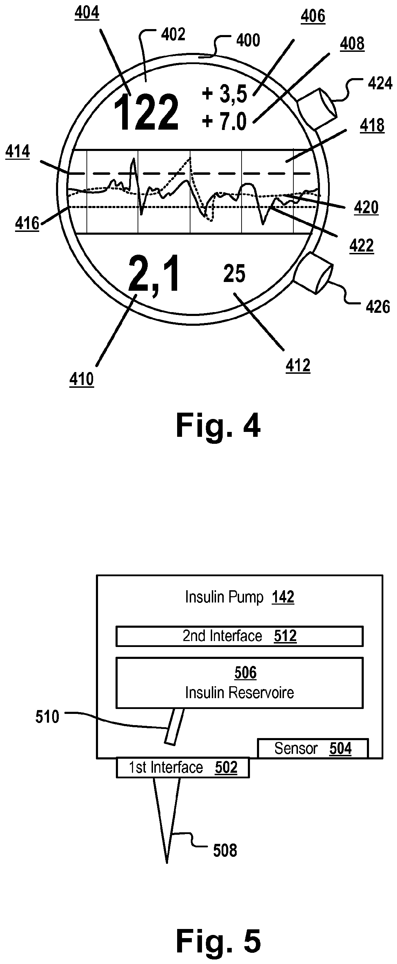

**Figure 4** is a schematic drawing of a display 402 of a smart watch 400.

According to preferred embodiments, the smart watch is communicatively coupled to a plurality of data collection units which comprise a continuous glucose monitoring device (CGM device). A CGM device is a device that measures glucose levels in real-time throughout the day and night. A tiny electrode called a glucose sensor is inserted under the skin to measure glucose levels in tissue fluid. It is connected to a transmitter that sends the information, typically via wireless radio frequency, to a monitoring and display device and/or to a smart phone or other portable computing device.

The smart watch comprises a client application program 114 as described already for figure 1. The GCM device can forward the measured blood glucose levels to the client application for enabling the client application to detect and notify a patient if the glucose level of the patient is reaching a high or low limit. The client application program of the smart watch is configured to repeatedly receive patient related measurement data from a CGM device coupled to the smart watch via Bluetooth or another wireless communication link. In addition, the smart watch is coupled to one or more additional data collection units, e.g. a thermometer or a gyroscope. The display shows a graphic user interface (GUI) generated by the client application program running on the smart watch.

The upper third of the GUI shows a number 404 which is indicative of a currently measured blood glucose level of the patient wearing the smart watch. In addition, the right side of the upper third of the GUI comprises a number 406 representing the blood glucose level having been measured five minutes ago and a number 408 representing the blood glucose level having been measured 15 minutes ago.

The central part of the GUI shows a dynamically and repeatedly updated plot 518. Each interval between two adjacent vertical auxiliary lines in the plot represents a time interval of one hour. The plot shows a profile 420 of the patient's pulse during the last few hours. In addition, the plot shows a profile 422 of the measured blood glucose level of the patient during the last few hours. The plot can optionally, in case additional data collection units are available, comprise further profiles, e.g. a profile 414 of the patient's total stress score or a profile 416 of the patient's body temperature.

The lower third of the GUI shows a number 410 which is indicative of a currently insulin level of the patient. In addition, the right side of the lower third of the GUI comprises a number 412 representing the measured currently active carbohydrates in the blood of the patient. For example, one unit insulin may be needed for 10 gram carbohydrates. Based on the currently measured insulin level 410 of the patient (2.1 units) and a currently measured blood glucose level 404 having a value of 122 units (e.g. gram/l blood), it can be predicted that there will be a particular glucose value after 1 hour (e.g. 8 units (e.g. g) carbohydrates per l blood).

Thus, each time a patient glances at his or her wrist, the patient may check his or her current physiologic state related to the blood glucose level. The smart watch may enable the patient to change the GUI into the typical GUI of a digital or analog clock, e.g. by pressing a button or by performing a wiping gesture over the display that may be implemented as touch screen.

The smart watch may comprise additional functions not related to the management of diabetes, e.g. messenger, e-mail and telecommunication functionalities, a camera, music player or other functions and respective interfaces and sensors.

According to some embodiments, the smart watch 400 is used as the portable battery powered computing device 102 described, for example, in figure 1. Hence, the smart watch may be used instead of a smartphone for diabetes management. According to other embodiments, the client application is a distributed application comprising a first and a second sub-application which are adapted to interact with each other for providing the functionality described already for the standalone client application program. The first sub-application is installed on the smartphone of the patient and the second sub-application is installed on the smart phone.

According to some embodiments, the smart watch further comprises a "sport now" button 424. The user may press the button 424 when he or she begins doing sports or another type of physical activity. The smart watch is configured such that the pressing of the button 424 shifts the desired ("target") value for blood sugar and reduces the factor for hydrocarbons in order to minimize the risk of hypo-glycemia by proactively reducing the active insulin amount - even before the client application or the sensors can automatically determine that the patient performs sporting activities. The algorithms than can determine the end of the sport mode by analyzing the other sensors.

According to some embodiments, the smart watch further comprises an "eat now" button 426. The user may press the eat now button 426 immediately when he or she begins eating. The eat now button sets one or more target values to a minimal value. For example, the target blood glucose is set to a low value.

Both buttons 424, 426 can be implemented as hardware buttons or can be realized by selectable GUI elements of a GUI displayed on a touch screen of the smart watch. The selection of any one of the buttons 424, 246 on the GUI can also be implemented via touch control gestures etc.

**Figure 5** is a block diagram of an insulin pump 142 assigned to a patient 104. The insulin pump comprises an insulin injection needle 508. The injection needle 508 is reversibly coupled to the insulin pump via interface 502. The needle 508 is connected to an insulin reservoir 506 via a flexible plastic duct 510. The interface 502 can be a flexible duct, e.g. a flexible duct of 0.5-40 cm, allowing relative movement between the needle and the insulin pump. The needle comprises a data carrier, e.g. matrix code as depicted in figure 6B or an RFID chip as depicted in figure 6A.

According to some embodiments, the insulin pump comprises a sensor 504 that is adapted to read data from the data carrier of the insulin injection needle 508 that is currently coupled to the insulin pump. For example, the sensor can be an optical scanning device, e.g. a camera, adapted for capturing an image of a two-dimensional code that is used as the data carrier of the needle. Alternatively, the sensor can be an RFID reader device adapted to reach data from an RFID chip attached to the needle. In some other embodiments, the sensor 504 adapted to read data from the data carrier may in addition or alternatively be a part of the portable computing device 102, thereby acting as one of the data collection units of the device 102.

The insulin pump comprises a further interface 512 adapted to establish a communication link, e.g. a Bluetooth connection, to an interface 109 of the portable computing device 102 assigned to the patient 104. The data that is read by the sensor 504 from the data carrier of the needle 508 is forwarded via the interface 512 to the client application 114. Hence, the sensor 504 is used as a data collection unit for providing patient related data to the client application.

In some embodiments, the sensor 504 can in addition be adapted to reach data from other consumables contained in the insulin pump 142. For example, the duct 510 and/or the replaceable insulin reservoir 506 can also comprise the data carriers, e.g. an optical code or RFID chip comprising some information on the manufacturer, expiration date, time and date of inserting or coupling the consumable to the insulin pump, the lot number, type ID being indicative of the type of consumable, and the like. The sensor can be configured to read data from all data carriers of all consumables contained in or attached to the insulin pump every time the insulin pump performs an injection. In addition, or alternatively, the sensor can be configured to read data from all a data carriers of all consumables contained in or attached to the insulin pump after predefined time intervals, e.g. every five minutes. Capturing and forwarding data related to the use and type of consumables of an insulin injection pump may be advantageous, because this information enables the client application and/or the server application to predict the time in the future when a particular patient will run out of a particular type of consumable. In some embodiments, the client application can comprise or can be operatively coupled to an automated ordering system that is configured to automatically order new consumables well ahead of the time the consumable is exhausted. In addition, a statistical analysis of health-related parameters of a plurality of patients in combination with an analysis of the production batch of the respectively used consumables may allow an early identification of defective product batches.

In some embodiments, the insulin pump is configured to send the data read from the data carrier of the needle and from other consumables, if any, to the coupled portable computing device. The client application program 114 and/or the server application program can be configured to generate a stop command in case the client application program or the server application program have detected that the needle (or any other consumable) is defective are short for any other reason not be used any more. The stop command is returned to the insulin pump 142. In response to receiving the stop command, the insulin pump prohibits any manual or automated injection of insulin by the insulin pump as long as the insulin injection needle currently coupled to the insulin pump remains in use. Optionally, in case the insulin pump comprises a graphical user interface and a display, the insulin pump may display a warning message that prompts the user to exchange the needle or the other consumable that was detected to be defective.

**Figure 6** shows two different insulin injection needles 600, 620 The insulin injection needles can optionally comprise an inner coating 602, e.g. a Teflon-based coating, to ease the residue-free injection of insulin through the needle. The needle is a flexible tube, e.g. a tube made of silicone or plastic. The needle is used to subcutaneously insert electrodes 604 which are part of or connected to a glucose sensor in a patient's body. The two electrodes 604 are subcutaneously integrated and are in contact with the tissue fluid or blood. The glucose level in a patient's blood can be determined by interpreting the resistance between the two electrodes 604 contained within or outside of the needle 600, 620.

The needle 600 depicted in **figure 6A** comprises a data carrier 608 in the form of an RFID chip comprising an electronic data storage medium. The chip can be connected to electrodes 604 for receiving impedance data being indicative of a current glucose level. In addition, the chip can be connected to a transmitting unit adapted to generate and/or amplify a signal that comprises the data stored in the data carrier and data being indicative of the current glucose level.

The transmitting unit (not shown) is adapted to generate a signal that is transported to the sensor. For example, the signal can be a radio signal (transported e.g. via RFID technique) or can be near field communication (NFC) signal, e.g. Bluetooth signal. NFC is a set of communication protocols that enable two electronic devices, one of which is usually a portable device such as a smartphone, to establish communication by bringing them within 4 cm of each other.

The signal received by the sender comprises the electric signal measured inside the body and/or the current glucose level computed therefrom, and preferably further comprises the information that is stored in the data carrier 608. The signal can be transmitted from the needle directly or indirectly to the client application. For example, , the electric signal measured inside the body and/or the current glucose level computed therefrom, and the additional information are communicated to a controller of the insulin pump first and then from the controller to the client application. The measured glucose level and the data contained in the data carriers are (actively or passively) transmitted to the client application using different protocols or communication interfaces.

The needle 600 comprises two electrodes 604 which are connected to a glucose meter and used for an impedance-based measuring of blood glucose level. The electrodes 604 are in addition connected to the data carrier 608 and used by a sensor 504 for reading out data from the data carrier. Hence, the electrodes 604 are dual-use electrodes. This may be beneficial because the costs related to the electrode material and to the attachment of electrodes on the needle surface may be reduced. In a further beneficial aspect, a needle is provided whose electrodes may be particularly robust against mechanical stress.

The needle 620 depicted in **figure 6B** comprises a data carrier having the form of an optically readable code, in particular a 2D code. Hence, the data carrier is an optical data carrier whose data can be read using an image acquisition device 504, e.g. a camera.

The data stored in the data carrier preferably comprises at least an identifier of the needle type and/or the lot number. Optionally, the data carrier comprises further data such as manufacturer, expiration date, etc. The lot number is an identifier of the product batch of a particular product, e.g. an insulin injection needle. A lot number is an identification number assigned to a particular quantity or lot of material from a single manufacturer. Lot numbers can typically be found on the outside of packaging. The lot number enables tracing of the constituent parts or ingredients as well as labor and equipment records involved in the manufacturing of a product. This enables manufacturers and other entities to perform quality control checks, calculate expiration dates, and issue corrections or recall information to subsets of their production output. It also gives consumers an identifier that they can use in contacting the manufacturer and researching the production of goods received.

The data contained in the data carrier 608, 622 of the sensor is actively transmitted to or is read out by a sensor, e.g. an RFID reader or an optical reading device. The sensor can be an integral part of the insulin pump. Alternatively, the sensor can be an integral part of the mobile battery powered computing device 102, e.g. a smart phone or a smart watch of a patient.

**Figure 7A** shows a bird's eye view of a system 700 comprising an insulin pump 142 and an insulin needle 600 which are both integrated on the same plaster 702. The insulin needle 600 comprises a data carrier 608, e.g. an RFID chip comprising some information on the manufacturer, expiration date, time and date of inserting or coupling the consumable to the insulin pump, the lot number, type ID being indicative of the type of consumable, and the like.

The insulin pump 142 can be a coin-sized, flat device that is pressed onto the skin of the patient (e.g. the skin of the upper arm). The insulin pump is coupled to or integrated in a plaster 702 that is used to fasten the pump and the needle to the skin.

The needle 600 is adapted to penetrate the skin and the tissue painlessly.

The system 700 further comprises a glucose sensor (not shown) that is adapted to receive current glucose measurement data from the electrodes 604 which continuously measure the concentration of sugar in the interstitial fluid of the skin and that is adapted to receive the data encoded in the data carrier 608 via a radio signal or a NFC link. The glucose sensor can be a microcontroller, e.g. a chip, connected to electrodes 604 via conductive wires for receiving current blood glucose levels and communicatively coupled to RFID chip 608 acting as the data carrier for receiving additional data, e.g. the lot number of the insulin needle 600. For example, the glucose sensor and the sensor 504 adapted for receiving/reading data from the data carrier can be implemented as a single, integrated sensor, e.g. a single sensor chip. Alternatively, the glucose sensor and the sensor 504 for receiving the data from the data carrier can be implemented as separate sensors, e.g. separate chips. The sensor 504 can be implemented as an RFID chip adapted to communicate both the glucose level data and the data stored in the data carrier 608 to a client application hosted in a mobile battery powered computing device 102. For example, the glucose measurement data and the data read from the data carrier can be transmitted from the sensor 504 to a data collection unit 138 of a smartphone 102 of a patient via a Bluetooth connection.

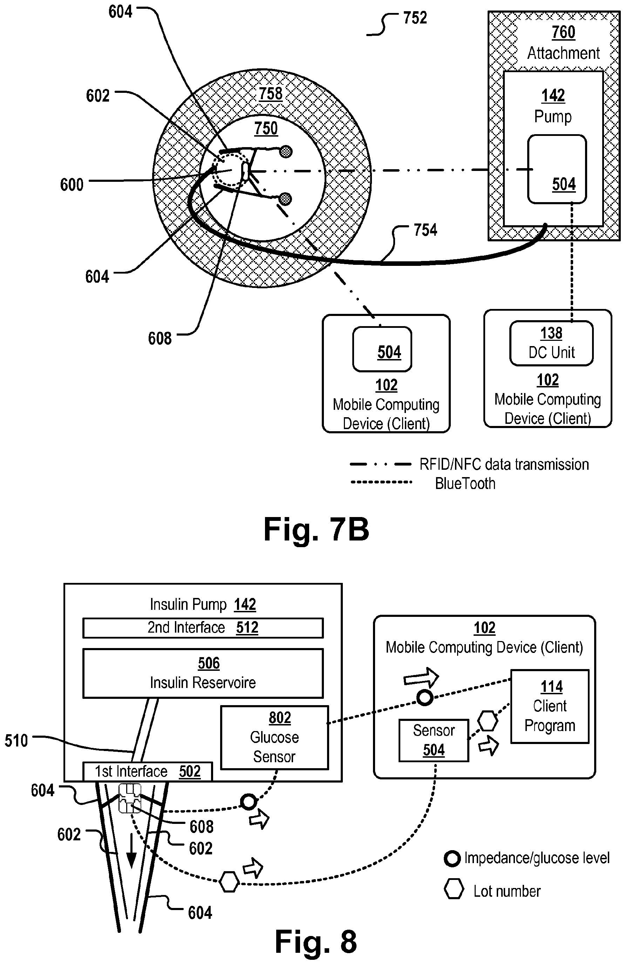

Figure 7B depicts an alternative of a system 752 comprising an insulin injection needle and an insulin pump. For example, the needle is attached to the human body via plaster 758 and the insulin pump is attached to another part of a patient's body or clothing via another attachment means 760, e.g. another plaster or a carrying strap or the like. The insulin pump 142 and the needle 600 are connected via a flexible duct 754 allowing a relative movement of the needle and the insulin pump 142. Attaching the needle and the insulin pump separately may have the advantage that the needle is more firmly fixed to the human body, because the plaster 758 does only have to hold the comparatively lightweight needle, not the heavy insulin pump and the reservoir.

The data contained in the data carrier 608 and the glucose measurement data obtained via the electrodes 604 may be transmitted e.g. via RFID technology to a sensor 504 that is an integral part of the insulin pump 142. Alternatively, the data contained in the data carrier 608 and the glucose measurement data obtained via the electrodes 604 may be transmitted e.g. via Bluetooth or a similar near field communication technology to a sensor 504 that is part of or coupled to a portable computing device 102 of the patient.

For example, the insulin pump 142 comprises an interface for receiving command data from the client application program 114, whereby the command data may specify a particular insulin bolus or base insulin rate to be injected by the insulin pump, whereby the bolus or base rate is computed by the client application program or the server application program as a function of the data collected by the data collection units as explained above.

Figure 8 depicts a portable computing device 102 assigned to a patient with diabetes. The portable computing device can be a smartphone, a smart watch or any other portable computing device, e.g. a portable computing device described already for other embodiments of the invention. The computing device 102 comprises a sensor 504 and a client application 114. The sensor 504 is adapted to read data from the data carrier of an insulin injection needle currently coupled to an insulin pump 142 as described herein for embodiments of the invention. The client application is interoperable with a server application. The portable computing device is operatively coupled to the insulin pump, e.g. by establishing a BlueTooth connection for receiving glucose measurement data from the glucose sensor 802. The glucose sensor 802 may receive or compute current glucose levels based on impedance measurements obtained from two electrodes 604. The sensor 504 is adapted to receive data stored in the data carrier 608, e.g. the lot number of the insulin needle currently coupled to the pump 142. The client application is configured for receiving data stored in the data carrier of the insulin needle via the sensor; sending the received data to the server application via a network for enabling the server application to compute - from the data of the data carrier of the insulin needle of the patient and from the data of the respective data carriers of a plurality of other insulin needles used by other patients - whether the currently coupled insulin needle has a manufacturing error; receiving from the server application a response message being indicative whether the currently coupled insulin needle has a manufacturing error; and selectively in case the response message indicates that currently coupled insulin needle has a manufacturing error, sending a stop command to the insulin pump, the stop command being adapted to prohibit any manual or automated injection of insulin by the insulin pump as long as the insulin injection needle currently coupled to the insulin pump remains in use.

**List of reference numerals**

| 100 | distributed computer system for diabetes management |
| 102 | portable computing device |
| 104 | diabetes patient |
| 106 | main memory |
| 108 | processors |
| 109 | interface(s) to data collection units |
| 110 | storage medium |
| 111 | network interface |
| 112 | display |
| 114 | client application program |
| 116 | prediction logic |
| 118 | prediction logic |
| 120 | prediction logic |
| 121 | network |
| 122 | request for performing prediction remotely |
| 123 | response comprising prediction result |
| 124 | current patient data |
| 126 | connectivity checker |
| 128 | calendar application |
| 130 | local patient history |
| 132 | plug-in of calendar application |
| 134 | data collection unit |
| 136 | data collection unit |
| 138 | data collection units |
| 140 | data collection unit (continuous glucose monitoring device) |
| 142 | insulin pump |
| 150 | server computer system |

| | |
|---|---|
| 152 | DBMS |
| 154 | database |
| 156 | patient registry |
| 158 | healthcare provider registry |
| 160 | remote history data of multiple patients |
| 162 | storage medium |
| 164 | sever application program |
| 165 | application server |
| 166 | main memory |
| 168 | statistical analysis module |
| 169 | network interface |
| 170 | prediction logic |
| 171 | processor |
| 172 | prediction logic |
| 174 | smart watch |
| 176 | smart phone |
| 178 | patient |
| 180 | patient |
| 182 | data source |
| 184 | data source |
| 186 | data source (continuous glucose monitoring device) |
| 190 | data source (insulin pump with sensor) |
| 192 | data collection unit |
| 194 | data collection unit (continuous glucose monitoring device) |
| 196 | data collection unit (insulin pump with sensor) |
| 198 | broadcasting service |
| 202-210 | steps |
| 302-310 | steps |
| 322-330 | steps |
| 400 | smart watch |
| 402 | display of smart watch |
| 404 | currently measured blood glucose level |
| 406 | blood glucose level measured five minutes ago |
| 408 | blood glucose level measured 15 minutes ago |
| 410 | currently active insulin |
| 412 | currently active carbohydrates |
| 414 | physiologic parameter (stress level) |
| 416 | environmental or physiologic parameter (temperature) |
| 418 | glucose level/pulse plot |
| 420 | pulse profile (dotted line) |
| 422 | blood glucose level profile (continuous line) |
| 424 | "sport now" button |
| 426 | "eat now" button |
| 502 | interface for coupling a needle to an insulin pump |
| 504 | sensor for receiving data from data carrier (RFID reader, camera) |
| 506 | insulin reservoir |
| 508 | insulin injection needle |
| 510 | duct |
| 600 | insulin injection needle |
| 602 | Teflon-coated inner surface of needle |

| | |
|---|---|
| 604 | electrodes |
| 608 | data carrier (RFID chip) |
| 620 | insulin injection needle |
| 622 | data carrier (matrix code) |
| 700 | system with insulin pump and needle integrated on one plaster |
| 702 | plaster |
| 704 | inner plate with needle and sensor |
| 752 | system with insulin separately attachable pump and needle |
| 754 | insulin duct |
| 758 | plaster |
| 802 | glucose sensor |

**Claims**

1. A system(100) for diabetes disease management, the system comprising:

   - a battery powered portable computing device (102, 174, 176) comprising a client application (114), the portable computing device being assigned to a diabetes patient (104), the portable computing device configured to be operatively coupled to a first and other data collection units (134, 136, 140, 132, 142; 192, 194, 196; 182, 184, 186, 190), each data collection unit being configured to collect data of a respective different data type, the data being related to the patient;
   - a server computer (150) comprising a server application (164), the server application being interoperable with the client application and implementing and maintaining a plurality of different prediction logics (116, 118, 120, 170, 172), each prediction logic being configured for remotely computing a prediction (123) of a future physiologic state of the patient and/or a treatment suggestion for the patient,
   - the client application being configured for:

      ◦ receive first data from the first data collection unit, the first data collection unit comprising a glucose meter currently coupled to the portable computing device; and
      ◦ upon receiving the first data, try to receive other data from the other data collection units currently coupled to the portable computing device,

   - the server application being configured for:

      ◦ receiving (322) current patient data (124) from the client application via a network (121), the received current patient data comprising a largest amount of the first data and the other data currently available as collected by the glucose meter and the other data collection units currently coupled to the portable computing device;
∘ identifying (324) the one or more data collection units having collected the current patient data;
∘ dynamically determining a number and type of the identified one or more data collection units;
∘ automatically identifying (326) and selecting a particular one out of the plurality of prediction logics as a function of at least the number and type of the identified one or more data collection units that is able to compute a prediction based on the largest amount of data currently available, the one identified prediction logic being adapted to use the totality of the received current patient data as input; and
∘ computing the prediction based on applying (328) the identified prediction logic at least on the received current patient data and on a history of the patient's patient data; and
∘ returning (330) the prediction to the client application via the network

wherein the client application is configured for: sending a command to an insulin pump (142) of the patient, the command causing the insulin pump to inject an amount of insulin to the patient that corresponds to a basal insulin rate specified in the prediction, wherein each of the other data collection units is selected from a group comprising:

- a motion tracking sensor;
- an ECG (electrocardiogram) device, the data collected by the ECG device being physiological measurement data, in particular an electrocardiogram and/or data values derived therefrom;
- a respirometer, the data collected by the respirometer being physiological measurement data, in particular a rate of oxygen and/or carbon dioxide exchange;
- a scale, the data collected by the scale being the body weight of the patient;
- a thermometer, the data collected by the thermometer being a patient-proximal temperature, the patient-proximal temperature being the body temperature of the patient or being the temperature of the air surrounding the patient;
- an insulin pump logging unit, the data collected by the insulin pump logging unit being the time and/or amount of insulin applied to the patient.

2. The system of claim 1, the server application being further configured for:

∘ receiving current environmental data from one or more data broadcast services (198) via the network, the current environmental data selectively comprising environmental parameter values currently measured in a geographic region where the portable computing de-vice is currently located; and/or
∘ receiving future environmental data from one or more data broadcast services (198) via the network, the future environmental data selectively comprising environmental parameter values predicted to be measured in the future in a geographic region where the portable computing device is currently located;

wherein the automated identification of the one out of the plurality of prediction logics is further performed as a function of the type of the received future or current environmental data.

3. The system of claim 2,
wherein the environmental data comprises a geographic-region-temperature measured or predicted to be measurable in the geographic region, the geographic region having a size of at least 1 km$^2$, preferably of at least 5 km$^2$; and/or
wherein the data collected by one of the data sources is a patient-proximal temperature, the patient-proximal temperature being the body temperature of the patient or being the temperature of the air surrounding the patient; and/or
wherein the one identified prediction logic is configured to compute a temperature-derived stress score as a function of at least the patient-proximal temperature and the geographic-region-temperature, the temperature-derived stress score being indicative of the physiologic stress level of the patient; and/or
wherein the one identified prediction logic is configured to remotely compute the prediction as a function of at least the current patient data, the history patient data of the patient and of the temperature-derived stress score.

4. The system of any one of the previous claims, wherein the history of the patient data used by the server application for the prediction is a remote copy of a local history of the patient's patient data, the local history being stored on the portable computing device, the remote history being stored on a storage medium accessible by the server computer, the client application and the server application being configured to repeatedly and fully automatically propagate changes in the local history of the patient's patient data to the remote history.

5. The system of any one of the previous claims, wherein the client application and/or the server application is further configured for:

- accessing data on the amount of consumables for insulin injection currently available to the patient;

- predicting the amount of consumables required by the patient in a future time interval as a function of at least the current patient data and the patient history;

- in response to determining that the amount of available consumables is below the amount of predicted, required consumables, automatically ordering new consumables from one or more suppliers or sending an invitation message to a health care provider to prescribe the new consumables.

6. A portable computing device (102, 174, 176) assigned to a diabetes patient (104), the portable computing device comprising:

- a client application (114);
- one or more interfaces (109) for reversibly coupling the client application operatively to a first and other different data collection units (134, 136, 138, 140, 142; 182-186, 190; 192-196), each data collection unit being configured to collect data of a respective different data type, the data being related to the patient;
- a network interface (111) configured to operatively couple the client application to a server application (164) running on a remote server (150);

wherein the client application is configured for locally computing a prediction of a future physiologic state of the patient and/or a treatment suggestion for the patient, wherein the client application implements and maintains a plurality of different prediction logics (116, 118, 120) configured to compute a prediction of a future physiologic state of the patient and/or a treatment suggestion for the patient, the client application being configured for:

receiving (304) a largest amount of first and other currently available patient data selectively from the data collection units currently being operatively coupled to the client application,

▪ the first data being received from the first data collection unit, the first data collection unit comprising a glucose meter currently coupled to the portable computing device,

▪ wherein the client application is further configured for, upon receiving the first data, trying to receive the other data from the other data collection units currently coupled to the portable computing device,

∘ identifying (324) the one or more data collection units having collected the current patient data

∘ dynamically determining a number and type of the identified one or more data collection units

∘ automatically identifying (306) and selecting a particular one out of the plurality of the prediction logics as a function of the number and type of the identified and currently coupled one or more data collection units that is able to compute a prediction based on the largest amount of data currently available, the one identified prediction logic being adapted to use the totality of the received patient data as input;

∘ applying (308) the identified local prediction logic on at least the current patient data and on a local history of the patient's patient data for computing the prediction; and

∘ sending a command to an insulin pump (142) of the patient, the command causing the insulin pump to inject an amount of insulin to the patient that corresponds to a basal insulin rate specified in the prediction,

∘ wherein each of the other data collection units is selected from a group comprising:

∘ a motion tracking sensor;

∘ an ECG (electrocardiogram) device, the data collected by the ECG device being physiological measurement data, in particular an electrocardiogram and/or data values derived therefrom;

∘ a respirometer, the data collected by the respirometer being physiological measurement data, in particular a rate of oxygen and/or carbon dioxide exchange;

∘ a scale, the data collected by the scale being the body weight of the patient;

∘ a thermometer, the data collected by the thermometer being a patient-proximal temperature, the patient-proximal temperature being the body temperature of the patient or being the temperature of the air surrounding the patient;

∘ an insulin pump logging unit, the data collected by the insulin pump logging unit being the time and/or amount of insulin applied to the patient.

7. The portable computing device of claim 6, wherein the portable computing device is a smartwatch (174, 400).

**Patentansprüche**

1. System(100) zur Verwaltung einer Diabeteserkrankung, wobei das System umfasst:

- eine batteriebetriebene, transportable Rechenvorrichtung (102, 174, 176), umfassend eine Clientanwendung (114), wobei die transportable Rechenvorrichtung einem Diabetespatienten (104) zugeordnet ist, wobei die transportable Rechenvorrichtung dazu ausgelegt ist, mit einer ersten und weiteren Datenerfassungseinheiten (134, 136, 140, 132, 142; 192, 194, 196; 182, 184, 186, 190) operativ gekoppelt zu sein, wobei jede Datenerfassungseinheit dazu ausgelegt ist, Daten eines entsprechenden anderen Datentyps zu erfassen, wobei sich die Daten auf den Patienten beziehen;

- einen Servercomputer (150), umfassend eine Serveranwendung (164), wobei die Serveranwendung in der Lage ist, mit der Clientanwendung zusammenzuarbeiten, und eine Vielzahl von unterschiedlichen Vorhersagelogiken (116, 118, 120, 170, 172) implementiert und unterhält, wobei jede Vorhersagelogik für ein entferntes Berechnen einer Vorhersage (123) eines zukünftigen physiologischen Zustands des Patienten und/oder eines Behandlungsvorschlags für den Patienten ausgelegt ist,

- wobei die Clientanwendung dazu ausgelegt ist:

  ◦ erste Daten von der ersten Datenerfassungseinheit zu empfangen, wobei die erste Datenerfassungseinheit ein Glukosemessgerät umfasst, das aktuell an die transportable Rechenvorrichtung gekoppelt ist; und

  ◦ bei Empfang der ersten Daten zu versuchen, weitere Daten von den weiteren Datenerfassungseinheiten zu empfangen, die aktuell an die transportable Rechenvorrichtung gekoppelt sind,

- wobei die Serveranwendung dazu ausgelegt ist:

  ◦ von der Clientanwendung über ein Netzwerk (121) aktuelle Patientendaten (124) zu empfangen (322), wobei die empfangenen aktuellen Patientendaten eine größte Menge der ersten Daten und der anderen Daten, die aktuell als vom Glukosemessgerät und den anderen aktuell an die transportable Rechenvorrichtung gekoppelten Datenerfassungseinheiten verfügbar sind, umfassen;

  ◦ die eine oder mehrere Datenerfassungseinheiten, die die aktuellen Patientendaten erfasst haben, zu identifizieren (324);

  ◦ eine Anzahl und einen Typ der identifizierten einen oder mehreren Datenerfassungseinheiten dynamisch zu bestimmen;

  ◦ aus der Vielzahl von Vorhersagelogiken als eine Funktion mindestens der Anzahl und des Typs der identifizierten einen oder mehreren Datenerfassungseinheiten automatisch eine bestimmte zu identifizieren (326) und auszuwählen, die in der Lage ist, basierend auf der größten Menge von aktuell verfügbaren Daten eine Vorhersage zu berechnen, wobei die eine identifizierte Vorhersagelogik dazu angepasst ist, die Gesamtheit der empfangenen aktuellen Patientendaten als Input zu nutzen; und

  ◦ die Vorhersage basierend auf dem Anwenden (328) der identifizierten Vorhersagelogik mindestens auf die empfangenen aktuellen Patientendaten und auf eine Historie der Patientendaten des Patienten zu berechnen; und

  ◦ über das Netzwerk die Vorhersage an die Clientanwendung zurückzugeben (330),

wobei die Clientanwendung dazu ausgelegt ist: einen Befehl an eine Insulinpumpe (142) des Patienten zu senden, wobei der Befehl die Insulinpumpe veranlasst, in den Patienten eine Menge von Insulin zu injizieren, die einer in der Vorhersage spezifizierten basalen Insulinrate entspricht,

wobei jede von den weiteren Datenerfassungseinheiten aus einer Gruppe ausgewählt ist, die umfasst:

- einen Bewegungsverfolgungssensor;
- eine EKG-Vorrichtung (Elektrokardiogrammvorrichtung), wobei die von der EKG-Vorrichtung erfassten Daten physiologische Messdaten sind, insbesondere ein Elektrokardiogramm und/oder davon abgeleitete Datenwerte;
- ein Respirometer, wobei die von dem Respirometer erfassten Daten physiologische Messdaten sind, insbesondere eine Rate von Sauerstoff- und/oder Kohlendioxidaustausch;
- eine Waage, wobei die von der Waage erfassten Daten das Körpergewicht des Patienten sind;
- ein Thermometer, wobei die von dem Thermometer erfassten Daten eine patientennahe Temperatur sind, wobei die patientennahe Temperatur die Körpertemperatur des Patienten ist oder die Temperatur der den Patienten umgebenden Luft ist;
- eine Insulinpumpenprotokollierungseinheit, wobei die von der Insulinpumpenprotokollierungseinheit erfassten Daten die Uhrzeit und/oder Menge von Insulin, das dem Patienten verabreicht wurde, sind.

2. System nach Anspruch 1, wobei die Serveranwendung ferner ausgelegt ist für:

  ◦ Empfangen aktueller Umweltdaten von einem oder mehreren Datenübertragungsdiensten (198) über das Netzwerk, wobei die aktuellen Umweltdaten wahlweise Umweltparameterwerte umfassen, die aktuell in einer geographischen Region gemessen werden, in der sich die transportable Rechenvorrichtung aktuell befindet; und/oder

◦ Empfangen zukünftiger Umweltdaten von einem oder mehreren Datenübertragungsdiensten (198) über das Netzwerk, wobei die zukünftigen Umweltdaten wahlweise Umweltparameterwerte umfassen, von denen vorhergesagt wird, dass sie zukünftig in einer geographischen Region gemessen werden, in der sich die transportable Rechenvorrichtung aktuell befindet;

wobei die automatisierte Identifizierung der einen aus der Vielzahl von Vorhersagelogiken ferner als eine Funktion des Typs der empfangenen zukünftigen oder aktuellen Umweltdaten ausgeführt wird.

3. System nach Anspruch 2,
wobei die Umweltdaten eine in der geographischen Region gemessene oder als messbar vorhergesagte Temperatur der geographischen Region umfassen, wobei die geographische Region eine Größe von mindestens 1 km$^2$, vorzugsweise von mindestens 5 km$^2$, hat; und/oder
wobei die von einer der Datenquellen erfassten Daten eine patientennahe Temperatur sind, wobei die patientennahe Temperatur die Körpertemperatur des Patienten ist oder die Temperatur der den Patienten umgebenden Luft ist; und/oder
wobei die eine identifizierte Vorhersagelogik dazu ausgelegt ist, einen temperaturabgeleiteten Belastungspunktwert als eine Funktion mindestens der patientennahen Temperatur und der Temperatur der geografischen Region zu berechnen, wobei der temperaturabgeleitete Belastungspunktwert bezeichnend für das physiologische Belastungsniveau des Patienten ist; und/oder
wobei die eine identifizierte Vorhersagelogik dazu ausgelegt ist, die Vorhersage entfernt als eine Funktion mindestens der aktuellen Patientendaten, der Historienpatientendaten des Patienten und des temperaturabgeleiteten Belastungspunktwerts zu berechnen.

4. System nach einem der vorangehenden Ansprüche, wobei die Historie der Patientendaten, die von der Serveranwendung für die Prognose genutzt werden, eine entfernte Kopie einer lokalen Historie der Patientendaten des Patienten ist, wobei die lokale Historie auf der transportablen Rechenvorrichtung gespeichert ist, wobei die entfernte Historie auf einem Speichermedium gespeichert ist, das durch den Servercomputer zugänglich ist, wobei die Clientanwendung und die Serveranwendung dazu ausgelegt sind, wiederholt und vollautomatisch Veränderungen in der lokalen Historie der Patientendaten des Patienten an die entfernte Historie zu übertragen.

5. System einem der vorangehenden Ansprüche, wobei die Clientanwendung
und/oder die Serveranwendung ferner ausgelegt ist für:

- das Zugreifen auf Daten zur Menge von Verbrauchsmitteln zur Insulininjektion, die dem Patienten aktuell verfügbar sind;
- das Prognostizieren der Menge von Verbrauchsmitteln, die von dem Patienten in einem zukünftigen Zeitintervall benötigt werden, als eine Funktion mindestens der aktuellen Patientendaten und der Patientenhistorie;
- in Reaktion auf das Feststellen, dass die Menge der verfügbaren Verbrauchsmittel unter der Menge der vorhergesagten benötigen Verbrauchsmittel liegt, automatisch Bestellen neuer Verbrauchsmittel von einem oder mehreren Lieferanten oder Senden einer Aufforderungsnachricht an einen Gesundheitsdienstleister, neue Verbrauchsmittel zu verschreiben.

6. Transportable Rechenvorrichtung (102, 174, 176), die einem Diabetespatienten (104) zugeordnet ist, wobei die transportable Rechenvorrichtung umfasst:

- eine Clientanwendung (114);
- eine oder mehrere Schnittstellen (109), um die Clientanwendung reversibel an eine erste und weitere andere Datenerfassungseinheiten (134, 136, 138, 140, 142; 182-186, 190; 192-196) zu koppeln, wobei jede Datenerfassungseinheit dazu ausgelegt ist, Daten eines jeweils anderen Datentyps zu erfassen, wobei sich die Daten auf den Patienten beziehen;
- eine Netzwerkschnittstelle (111), dazu ausgelegt, die Clientanwendung operativ an eine Serveranwendung (164), die auf einem entfernten Server (150) läuft, zu koppeln;

wobei die Clientanwendung ausgelegt ist für ein lokales Berechnen einer Vorhersage eines zukünftigen physiologischen Zustands des Patienten und/oder eines Behandlungsvorschlags für den Patienten, wobei die Clientanwendung eine Vielzahl unterschiedlicher Vorhersagelogiken (116, 118, 120), die dazu ausgelegt sind, eine Prognose eines zukünftigen physiologischen Zustands des Patienten und/oder einen Behandlungsvorschlag für den Patienten zu berechnen, umfasst, wobei die Clientanwendung dazu ausgelegt ist:
eine größte Menge erster und anderer aktuell verfügbarer Patientendaten wahlweise von den Datenerfassungseinheiten zu empfangen (304), die operativ an die Clientanwendung gekoppelt sind,

■ wobei die ersten Daten von der ersten Datenerfassungseinheit empfangen werden, wobei die erste Datenerfassungseinheit ein Glukosemessgerät umfasst, das aktuell an die transportable Rechenvorrichtung gekoppelt ist,

▪ wobei die Clientanwendung ferner dazu ausgelegt ist, bei Empfang der ersten Daten zu versuchen, die weiteren Daten von den weiteren Datenerfassungseinheiten zu empfangen, die aktuell an die transportable Rechenvorrichtung gekoppelt sind,

◦ die eine oder mehrere Datenerfassungseinheiten, die die aktuellen Patientendaten erfasst haben, zu identifizieren (324)

◦ eine Anzahl und einen Typ der identifizierten einen oder mehreren Datenerfassungseinheiten dynamisch zu bestimmen

◦ aus der Vielzahl von Vorhersagelogiken als eine Funktion der Anzahl und des Typs der identifizierten und aktuell gekoppelten einen oder mehreren Datenerfassungseinheiten automatisch eine bestimmte zu identifizieren (306) und auszuwählen, die in der Lage ist, basierend auf der größten Menge von aktuell verfügbaren Daten eine Vorhersage zu berechnen, wobei die eine identifizierte Vorhersagelogik dazu angepasst ist, die Gesamtheit der empfangenen Patientendaten als Input zu nutzen;

◦ die identifizierte lokale Vorhersagelogik mindestens auf die aktuellen Patientendaten und auf eine Historie der Patientendaten des Patienten zur Berechnung der Vorhersage anzuwenden (308); und

◦ einen Befehl an eine Insulinpumpe (142) des Patienten zu senden, wobei der Befehl die Insulinpumpe veranlasst, in den Patienten eine Menge von Insulin zu injizieren, die einer in der Vorhersage spezifizierten basalen Insulinrate entspricht,

◦ wobei jede von den weiteren Datenerfassungseinheiten aus einer Gruppe ausgewählt ist, die umfasst:

◦ einen Bewegungsverfolgungssensor;
◦ eine EKG-Vorrichtung (Elektrokardiogrammvorrichtung), wobei die von der EKG-Vorrichtung erfassten Daten physiologische Messdaten sind, insbesondere ein Elektrokardiogramm und/oder davon abgeleitete Datenwerte;
◦ ein Respirometer, wobei die von dem Respirometer erfassten Daten physiologische Messdaten sind, insbesondere eine Rate von Sauerstoff- und/oder Kohlendioxidaustausch;
◦ eine Waage, wobei die von der Waage erfassten Daten das Körpergewicht des Patienten sind;
◦ ein Thermometer, wobei die von dem Thermometer erfassten Daten eine patientennahe Temperatur sind, wobei die patientennahe Temperatur die Körpertemperatur des Patienten ist oder die Temperatur der den Patienten umgebenden Luft ist;
◦ eine Insulinpumpenprotokollierungseinheit, wobei die von der Insulinpumpenprotokollierungseinheit erfassten Daten die Uhrzeit und/oder Menge von Insulin, das dem Patienten verabreicht wurde, sind.

7. Transportable Rechenvorrichtung nach Anspruch 6, wobei die transportable Rechenvorrichtung eine Smartwatch (174, 400) ist.

**Revendications**

1. Système (100) permettant la gestion des maladies du diabète, le système comprenant :

- un dispositif informatique portable (102, 174, 176) alimenté par batterie comprenant une application client (114), le dispositif informatique portable étant associé à un patient diabétique (104), le dispositif informatique portable étant conçu pour être couplé fonctionnellement à une première unité et à d'autres unités de collecte de données (134, 136, 140, 132, 142 ; 192, 194, 196 ; 182, 184, 186, 190), chaque unité de collecte de données étant conçue pour collecter des données d'un type de données différent respectif, les données concernant le patient ;
- un ordinateur serveur (150) comprenant une application serveur (164), l'application serveur étant inter-opérationnelle avec l'application client, et mettant en oeuvre et maintenant une pluralité de logiques de prédiction (116, 118, 120, 170, 172) différentes, chaque logique de prédiction étant conçue pour calculer à distance une prédiction (123) d'un état physiologique futur du patient et/ou une suggestion de traitement pour le patient,
- l'application client étant conçue pour :

◦ recevoir des premières données à partir de la première unité de collecte de données, la première unité de collecte de données comprenant un glucose-mètre couplé actuellement au dispositif informatique portable ; et
◦ lors de la réception des premières données, essayer de recevoir d'autres données à partir d'autres unités de collecte de données couplées actuellement au dispositif informatique portable,

- l'application serveur étant conçue pour :

○ recevoir (322) des données de patient (124) actuelles à partir de l'application client par le biais d'un réseau (121), les données de patient actuelles reçues comprenant une quantité plus importante de premières données et des autres données disponibles actuellement telles que collectées par le glucose-mètre et les autres unités de collecte de données actuellement couplées au dispositif informatique portable ;

○ identifier (324) l'unité ou les unités de collecte de données ayant récolté les données du patient actuelles ;

○ déterminer de manière dynamique un nombre et un type de l'unité ou des unités de collecte de données identifiées ;

○ identifier automatiquement (326) et sélectionner une logique particulière parmi la pluralité des logiques de prédiction en fonction de l'au moins un nombre et type de l'unité ou des unités de collecte de données identifiées, qui est capable de calculer une prédiction sur la base de la plus grande quantité de données disponibles actuellement, la logique de prédiction identifiée étant adaptée pour utiliser la totalité des données de patient actuelles reçues en tant qu'entrée ; et

○ calculer la prédiction basée en appliquant (328) la logique de prédiction identifiée au moins aux données de patient actuelles reçues et sur l'historique des données de patient du patient ; et

○ retourner (330) la prédiction à l'application client par le biais du réseau

où l'application client est conçue pour : envoyer une commande à une pompe à insuline (142) du patient, la commande faisant en sorte que la pompe à insuline injecte une quantité d'insuline au patient qui correspond à un taux d'insuline basal spécifié dans la prédiction,

où chacune des autres unités de collecte de données est choisie dans un groupe comprenant :

- un capteur détecteur de mouvement ;
- un dispositif d'EGC (électrocardiogramme), les données collectées par le dispositif d'EGC étant des données de mesure physiologiques, en particulier un électrocardiogramme et/ou des valeurs de données en étant dérivées ;
- un respiromètre, les données collectées par le respiromètre étant des données de mesure physiologiques, en particulier un taux d'oxygène et/ou d'échange de dioxyde de carbone ;
- un barème, les données collectées par le barème étant le poids corporel du patient ;
- un thermomètre, les données collectées par le thermomètre étant une température proximale de patient, la température proximale de patient étant la température corporelle du patient ou étant la température de l'air entourant le patient ;
- une unité de consignation de pompe à insuline, les données collectées par l'unité de consignation de la pompe à insuline étant l'heure et/ou la quantité d'insuline appliquée au patient.

2. Système selon la revendication 1, l'application serveur étant en outre conçue pour :

○ recevoir des données environnementales actuelles à partir d'un ou de plusieurs services de diffusion de données (198) par le biais du réseau, les données environnementales actuelles comprenant sélectivement des valeurs de paramètres environnementaux mesurées actuellement dans une région géographique où le dispositif informatique portable est actuellement situé ; et/ou

○ recevoir des données environnementales futures à partir d'un ou de plusieurs services de diffusion de données (198) par le biais du réseau, les données environnementales futures comprenant sélectivement des valeurs de paramètres environnementaux prédites pour être mesurées dans le futur dans une région géographique où le dispositif informatique portable est actuellement situé ;

où l'identification automatisée de la logique parmi la pluralité des logiques de prédiction est en outre effectuée en fonction du type de la donnée environnementale future ou actuelle reçue.

3. Système selon la revendication 2,
dans lequel la donnée environnementale comprend une température de région géographique mesurée ou prédite d'être mesurée dans la région géographique, la région géographique ayant une taille d'au moins 1 km², de préférence d'au moins 5 km² ; et/ou
dans lequel la donnée collectée par une des sources de données est une température proximale de patient, la température proximale de patient étant la température corporelle du patient ou étant la température de l'air entourant le patient ; et/ou
dans lequel la logique de prédiction identifiée est conçue pour calculer un score de stress dérivé de la température en tant que fonction de l'au moins une température proximale de patient et de la température de région géographique, le score de stress dérivé de la température étant indicatif du niveau de stress physiologique du patient ; et/ou
dans lequel la logique de prédiction identifiée est conçue pour calculer à distance la prédiction en fonction des au moins données de patient actuelles, de l'historique des données de patient du patient et du score de stress dérivé de la température.

**4.** Système selon l'une quelconque des revendications précédentes, dans lequel l'historique des données du patient utilisé par l'application serveur pour la prédiction est une copie à distance d'un historique local des données de patient du patient, l'historique local étant stocké sur le dispositif informatique portable, l'historique à distance étant stocké sur un support de stockage accessible par l'ordinateur serveur, l'application client et l'application serveur étant conçues pour propager de manière répétée et totalement automatique des variations dans l'historique local des données de patient du patient vers l'historique à distance.

**5.** Système selon l'une quelconque des revendications précédentes, dans lequel l'application client et/ou l'application serveur sont en outre conçues pour :

    - avoir accès à des données concernant la quantité de consommables pour l'injection d'insuline actuellement disponible pour le patient ;
    - prédire la quantité de consommables nécessaire pour le patient dans un intervalle de temps futur en fonction des au moins données de patient actuelles et de l'historique du patient ;
    - en réponse à la détermination que la quantité de consommables disponibles est en-dessous de la quantité de consommables prédite, nécessaire, commander automatiquement de nouveaux consommables provenant d'un ou de plusieurs fournisseurs ou envoyer un message d'invitation à un fournisseur de soins de santé pour qu'il prescrive des nouveaux consommables.

**6.** Dispositif informatique portable (102, 174, 176) associé à un patient diabétique (104), le dispositif informatique portable comprenant :

    - une application client (114) ;
    - une ou plusieurs interfaces (109) pour coupler de manière réversible l'application client de manière fonctionnelle à une première unité et à d'autres unités de collecte de données (134, 136, 138, 140, 142 ; 182 à 186, 190 ; 192 à 196) différentes, chaque unité de collecte de données étant conçue pour récolter des données d'un type respectif de données différentes, les données étant en relation avec le patient ;
    - une interface réseau (111) conçue pour coupler fonctionnellement l'application client à une application serveur (164) fonctionnant sur un serveur à distance (150) ;

où l'application client est conçue pour calculer localement une prédiction d'un état physiologique futur du patient et/ou une suggestion de traitement pour le patient, où l'application client met en oeuvre et maintient une pluralité de logiques de prédiction (116, 118, 120) différentes conçues pour calculer une prédiction d'un état physiologique futur du patient et/ou une suggestion de traitement pour le patient, l'application client étant conçue pour :
recevoir (304) une quantité la plus importante de premières et d'autres données de patient disponibles actuellement étant couplées à l'application client,

    ■ la première donnée étant reçue à partir de la première unité de collecte de données, la première unité de collecte de données comprenant un glucose-mètre relié actuellement au dispositif informatique portable,
    ■ où l'application client est en outre conçue pour, lors de la réception des premières données, essayer de recevoir les autres données à partir des autres unités de collecte de données actuellement couplées au dispositif informatique portable,
    ∘ identifier (324) l'unité ou les unités de collecte de données ayant récolté les données du patient actuelles ;
    ∘ déterminer de manière dynamique un nombre et un type de l'unité ou des unités de collecte de données identifiées ;
    ∘ identifier automatiquement (306) et sélectionner une logique particulière parmi la pluralité des logiques de prédiction en fonction de l'au moins un nombre et type de l'unité ou des unités de collecte de données identifiées, qui est capable de calculer une prédiction sur la base d'une grande quantité de données disponibles actuellement, la logique de prédiction identifiée étant adaptée pour utiliser la totalité des données de patient actuelles reçues en tant qu'entrée ;
    ∘ appliquer (308) la logique de prédiction locale identifiée à l'au moins une donnée de patient actuelle et à l'historique local des données de patient du patient pour calculer la prédiction ; et
    ∘ envoyer une commande à une pompe à insuline (142) du patient, la commande faisant en sorte que la pompe à insuline injecte une quantité d'insuline au patient qui correspond à un taux d'insuline basal spécifié dans la prédiction,
    ∘ où chacune des autres unités de collecte de données est choisie dans un groupe comprenant :

      ∘ un capteur détecteur de mouvement ;
      ∘ un dispositif d'EGC (électrocardiogramme), les données collectées par le dispositif d'EGC étant des données de mesure physiologiques, en particulier un électrocardiogramme et/ou des valeurs de données en étant dérivées ;
      ∘ un respiromètre, les données collectées par le respiromètre étant des données de mesure physiologiques, en particulier un taux d'oxygène et/ou un échange de dioxyde de carbone ;

◦ un barème les données collectées par le barème étant le poids corporel du patient ;

◦ un thermomètre, les données collectées par le thermomètre étant une température proximale de patient, la température proximale de patient étant la température corporelle du patient ou étant la température de l'air entourant le patient ;

° une unité de consignation de pompe à insuline, les données collectées par l'unité de consignation de la pompe à insuline étant l'heure et/ou la quantité d'insuline appliquée au patient.

7. Dispositif informatique portable selon la revendication 6, où le dispositif informatique portable est une montre intelligente (174, 400).

**Fig. 1**

```
┌─────────────────────────────────────────────────────────────────┐
│ Repeatedly checking if a network connection to the server computer is available │ ⌐ 202
└─────────────────────────────────────────────────────────────────┘
                                │
                                ▼
                    ╱─────────────────────╲  ⌐ 204
                    ╲   Connection available?  ╱
                         │               │
                        NO              YES
                         │               │
                         ▼               ▼
          ┌──────────────────┐    ┌──────────────────────────┐
          │ Performing a local │⌐212│   Sending a request for    │ ⌐ 206
          │ computation of the │    │ performing the remote      │
          │    prediction      │    │ computation of the prediction to │
          └──────────────────┘    │   the server application   │
                         │         └──────────────────────────┘
                         │                      │
                         │                      ▼
                         │         ┌──────────────────────────┐
                         │         │ Receiving the prediction from │ ⌐ 208
                         │         │    the server application    │
                         │         └──────────────────────────┘
                         │                      │
                         ▼                      ▼
          ┌─────────────────────────────────────────────────────┐
          │              Outputting the prediction               │ ⌐ 210
          └─────────────────────────────────────────────────────┘
```

# Fig. 2

Client Side

```
┌─────────────────────────────────────────────────────────────────┐
│ Identifying one or more data collection units currently coupled to the client │ ⌐ 302
│                          application                              │
└─────────────────────────────────────────────────────────────────┘
                                │
                                ▼
┌─────────────────────────────────────────────────────────────────┐
│ Receiving current patient data selectively from currently coupled data collection │ ⌐ 304
│                             units                                 │
└─────────────────────────────────────────────────────────────────┘
                                │
                                ▼
┌─────────────────────────────────────────────────────────────────┐
│ Automatically identifying the data collection units having collected the current │ ⌐ 305
│                         patient data                             │
└─────────────────────────────────────────────────────────────────┘
                                │
                                ▼
┌─────────────────────────────────────────────────────────────────┐
│ Automatically identifying the one out of a plurality of prediction logics which is │ ⌐ 306
│       adapted to use the totality of the received patient data as input          │
└─────────────────────────────────────────────────────────────────┘
                                │
                                ▼
┌─────────────────────────────────────────────────────────────────┐
│ Applying the identified local prediction logic on at least the current patient data │ ⌐ 308
│ and on a local history of the patient's patient data for computing the prediction  │
└─────────────────────────────────────────────────────────────────┘
                                │
                                ▼
┌─────────────────────────────────────────────────────────────────┐
│                       Outputting the prediction                  │ ⌐ 310
└─────────────────────────────────────────────────────────────────┘
```

# Fig. 3A

Server Side

Receiving current patient data from a client application via a network ⌐ 322

Automatically identifying the data collection units having collected the current patient data ⌐ 324

Automatically identifying the one out of a plurality of prediction logics which is adapted to use the totality of the received patient data as input ⌐ 326

Applying the identified remote prediction logic on at least the current patient data and on a remote history of the patient's patient data for computing the prediction ⌐ 328

Returning the prediction ⌐ 330

# Fig. 3B

**Fig. 4**

**Fig. 5**

600

608

604

604

602

602

**Fig. 6A**

620

604

622

604

602

602

**Fig. 6B**

604

700

702

602

704

600

504

754

142

604

102
Mobile Computing
Device (Client)

138
DC Unit

608

— · · — RFID/NFC data transmission
------------ BlueTooth

**Fig. 7A**

604

752

758

760
Attachment

602

142
Pump

750

504

600

604

608

754

504

102
Mobile Computing
Device (Client)

138
DC Unit

102
Mobile Computing
Device (Client)

— · · — RFID/NFC data transmission
············· BlueTooth

## Fig. 7B

Insulin Pump **142**

2nd Interface **512**

**506**
Insulin Reservoire

**802**
Glucose
Sensor

**102**
Mobile Computing Device (Client)

**114**
Client
Program

Sensor
**504**

510

1st Interface **502**

604

608

602

602

604

○ Impedance/glucose level

⬡ Lot number

## Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140012511 A1
- US 2017049963 A1, Varsavsky Andrea
- US 2017311903 A1, Davis Anna Leigh